# EUROPEAN PATENT APPLICATION

(11) **EP 1 241 255 A1**
(43) Date of publication of application: **18.09.2002**
(21) Application number: 00981761.0
(22) Date of filing: 15.12.2000
(51) Int. Cl.: C12N 15/12, C12N 1/21, C12N 9/00, C07K 14/47, C12P 21/02, C12Q 1/68, G01N 33/15, G01N 33/50, G01N 33/53, G01N 33/566, G01N 33/574, G01N 33/577, A61K 31/711, A61K 38/43, A61K 39/395, A61K 45/00, A61K 48/00

(54) **NOVEL HUMAN RNA HELICASE, HELICAIN**

(30) Priority: 16.12.1999 JP 35740699
(71) Applicant: CHUGAI SEIYAKU KABUSHIKI KAISHA, Tokyo, 115-8543 (JP)
(72) Inventor: SUGIHARA, Takashi, Sweatmaki 101, 31-3747,, Aomori 033-0033 (JP); WADHWA, Renu, Chugai Res. Inst. for Med. Scien, In, Ibaraki 300-4101 (JP)
(74) Representative: VOSSIUS & PARTNER
(86) International application number: JP0008908
(87) International publication number: WO01044470

(57) **Abstract**

Novel RNA helicase genes of human origin, Helicain A, B, and C, were isolated. The expression of these genes was significantly elevated in thyroid gland and cancer cells. Helicain proteins and genes encoding them can be used as tools for examination and development of pharmaceuticals for diseases, such as cancer.

SEQUENCE LISTING

<110> Chugai Seiyaku Kabushiki Kaisha

<120> Novel human RNA helicase, Helicain

<130> C2-114PCT

<140>

<141>

<150> JP 1999-357406

<151> 1999-12-16

<160> 20

<170> PatentIn Ver. 2.0

<210> 1

<211> 1387

<212> DNA

<213> Homo sapiens

<220>

<221> CDS

<222> (154)..(1110)

<400> 1

aaaaaaggac agcgagcggg gagccccgag gaccccctt tccctgccca gggattgagc 60

tccagagcct gttagggagc tacccagaca ggctttagtt cctaaagtgc tgaggaaacg 120

cacggggcgg taaaggggag gctcgtcggt gag atg gcg ccg gat ctc gct tct 174
Met Ala Pro Asp Leu Ala Ser
1               5

cag agg cat tca gag agc ttc ccc agc gtg aac tct cgg ccg aat gta 222
Gln Arg His Ser Glu Ser Phe Pro Ser Val Asn Ser Arg Pro Asn Val
        10                  15                  20

att ttg cca ggc cgc gag gga cgc aga gag ggg ctg cca cca ggc ggg 270
Ile Leu Pro Gly Arg Glu Gly Arg Arg Glu Gly Leu Pro Pro Gly Gly
    25                  30                  35

ggc acg cgc ggt agt ctt gtc cct acc cgc ccg gtg ccg ccg tcc ccg 318
Gly Thr Arg Gly Ser Leu Val Pro Thr Arg Pro Val Pro Pro Ser Pro
40                  45                  50                  55

gct ccc ctc ggg acg agt ccg tac agc tgg agc cgt tcc gga ccc gga 366
Ala Pro Leu Gly Thr Ser Pro Tyr Ser Trp Ser Arg Ser Gly Pro Gly
                60                  65                  70

agg ggc ggt ggc gca ggg tcc tcg cgc gtc cca cgt ggg gtc ccg ggc 414
Arg Gly Gly Gly Ala Gly Ser Ser Arg Val Pro Arg Gly Val Pro Gly
            75                  80                  85

cca gca gtc tgc gca ccg ggc tcg ctg ctg cac cac gcg tca ccc acg 462
Pro Ala Val Cys Ala Pro Gly Ser Leu Leu His His Ala Ser Pro Thr
        90                  95                  100

cag acc atg gca gcc gcc gac ggt tcg ctc ttc gac aac ccc agg acg 510
Gln Thr Met Ala Ala Ala Asp Gly Ser Leu Phe Asp Asn Pro Arg Thr
    105                 110                 115

ttc tcc aga cgt ccc cca gcc cag gcg agt cgg caa gca aag gct acg 558
Phe Ser Arg Arg Pro Pro Ala Gln Ala Ser Arg Gln Ala Lys Ala Thr
120                 125                 130                 135

aaa aga aaa tac caa gca tcc agt gag gct ccc cca gcg aaa cgg agg 606
Lys Arg Lys Tyr Gln Ala Ser Ser Glu Ala Pro Pro Ala Lys Arg Arg
                140                 145                 150

aac gaa act tca ttt ctc cca gcc aag aaa act agt gtt aaa gaa act 654
Asn Glu Thr Ser Phe Leu Pro Ala Lys Lys Thr Ser Val Lys Glu Thr
            155                 160                 165

cag agg act ttt aag ggg aac gca caa aaa atg ttt tct cca aag aag 702
Gln Arg Thr Phe Lys Gly Asn Ala Gln Lys Met Phe Ser Pro Lys Lys

        170                 175                 180

cat tcg gtt agc aca agt gat aga aac cag gag gag aga cag tgc att 750
His Ser Val Ser Thr Ser Asp Arg Asn Gln Glu Glu Arg Gln Cys Ile
    185                 190                 195

aag act tca tca ctg ttt aaa aac aac cct gac att cca gaa ctc cac 798
Lys Thr Ser Ser Leu Phe Lys Asn Asn Pro Asp Ile Pro Glu Leu His
200                 205                 210                 215

aga cct gtg gta aag cag gtg caa gaa aaa gtg ttt act tca gct gct 846
Arg Pro Val Val Lys Gln Val Gln Glu Lys Val Phe Thr Ser Ala Ala
                220                 225                 230

ttt cat gag ctg ggc ctc cac cca cat tta att tcc aca ata aat acg 894
Phe His Glu Leu Gly Leu His Pro His Leu Ile Ser Thr Ile Asn Thr
            235                 240                 245

gtc tta aaa atg tct agt atg acc agt gtt cag aag caa agt att cct 942
Val Leu Lys Met Ser Ser Met Thr Ser Val Gln Lys Gln Ser Ile Pro
        250                 255                 260

gtg ttg ctg gaa ggc aga gat gct ctc gtg aga tcc cag acg ggc tca 990
Val Leu Leu Glu Gly Arg Asp Ala Leu Val Arg Ser Gln Thr Gly Ser
    265                 270                 275

ggt aaa act ctt gcc tat tgc atc cct gtg gtc cag tcc ctt caa gca 1038
Gly Lys Thr Leu Ala Tyr Cys Ile Pro Val Val Gln Ser Leu Gln Ala
280                 285                 290                 295

atg gag tca aaa ata cag gta tta ctg tta tcc aca ttt tac gaa gaa 1086
Met Glu Ser Lys Ile Gln Val Leu Leu Leu Ser Thr Phe Tyr Glu Glu
                300                 305                 310

gaa cag agg ctt agg aaa gtt aaa taaccaaggc catgcagcaa gtaatggagg 1140
Glu Gln Arg Leu Arg Lys Val Lys
            315

aactggattt ctacctcagt tctgtttggc tgcagagacc aagctcctta ctcttgtgtg 1200

aagacttaca gtgaaggtta attcctggct cctgtgctat gtaggggcca tgcatctgtg 1260

tcttccacag tacctaacgt gggacctttt acagaatggt cactcaataa aggtgatttg 1320

aatttgcaaa aaaaaaaaaa aaaaaaaaaa aaaaaaaaaa aaaaaaaaaa aaaaaaaaaa 1380

aaaaaaa 1387

<210> 2
<211> 319
<212> PRT

<213> Homo sapiens

<400> 2

Met Ala Pro Asp Leu Ala Ser Gln Arg His Ser Glu Ser Phe Pro Ser
1               5                   10                  15

Val Asn Ser Arg Pro Asn Val Ile Leu Pro Gly Arg Glu Gly Arg Arg
            20                  25                  30

Glu Gly Leu Pro Pro Gly Gly Gly Thr Arg Gly Ser Leu Val Pro Thr
        35                  40                  45

Arg Pro Val Pro Pro Ser Pro Ala Pro Leu Gly Thr Ser Pro Tyr Ser
    50                  55                  60

Trp Ser Arg Ser Gly Pro Gly Arg Gly Gly Gly Ala Gly Ser Ser Arg
65                  70                  75                  80

Val Pro Arg Gly Val Pro Gly Pro Ala Val Cys Ala Pro Gly Ser Leu
                85                  90                  95

Leu His His Ala Ser Pro Thr Gln Thr Met Ala Ala Ala Asp Gly Ser
            100                 105                 110

Leu Phe Asp Asn Pro Arg Thr Phe Ser Arg Arg Pro Pro Ala Gln Ala
        115                 120                 125

Ser Arg Gln Ala Lys Ala Thr Lys Arg Lys Tyr Gln Ala Ser Ser Glu
    130                 135                 140
Ala Pro Pro Ala Lys Arg Arg Asn Glu Thr Ser Phe Leu Pro Ala Lys
145                 150                 155                 160
Lys Thr Ser Val Lys Glu Thr Gln Arg Thr Phe Lys Gly Asn Ala Gln
                165                 170                 175
Lys Met Phe Ser Pro Lys Lys His Ser Val Ser Thr Ser Asp Arg Asn
            180                 185                 190
Gln Glu Glu Arg Gln Cys Ile Lys Thr Ser Ser Leu Phe Lys Asn Asn
        195                 200                 205
Pro Asp Ile Pro Glu Leu His Arg Pro Val Val Lys Gln Val Gln Glu
    210                 215                 220
Lys Val Phe Thr Ser Ala Ala Phe His Glu Leu Gly Leu His Pro His
225                 230                 235                 240
Leu Ile Ser Thr Ile Asn Thr Val Leu Lys Met Ser Ser Met Thr Ser
                245                 250                 255
Val Gln Lys Gln Ser Ile Pro Val Leu Leu Glu Gly Arg Asp Ala Leu

            260                 265                 270

Val Arg Ser Gln Thr Gly Ser Gly Lys Thr Leu Ala Tyr Cys Ile Pro

        275                 280                 285

Val Val Gln Ser Leu Gln Ala Met Glu Ser Lys Ile Gln Val Leu Leu

    290                 295                 300

Leu Ser Thr Phe Tyr Glu Glu Glu Gln Arg Leu Arg Lys Val Lys

305                 310                 315

<210> 3

<211> 3283

<212> DNA

<213> Homo sapiens

<220>

<221> CDS

<222> (154)..(2706)

<400> 3

aaaaaaggac agcgagcggg gagccccgag gacccccttt tccctgccca gggattgagc 60

tccagagcct gttagggagc tacccagaca ggctttagtt cctaaagtgc tgaggaaacg 120

cacggggcgg taaaggggag gctcgtcggt gag atg gcg ccg gat ctc gct tct 174

1               5

cag agg cat tca gag agc ttc ccc agc gtg aac tct cgg ccg aat gta 222

        10                  15                  20

att ttg cca ggc cgc gag gga cgc aga gag ggg ctg cca cca ggc ggg 270

    25                  30                  35

ggc acg cgc ggt agt ctt gtc cct acc cgc ccg gtg ccg ccg tcc ccg 318

40                  45                  50                  55

gct ccc ctc ggg acg agt ccg tac agc tgg agc cgt tcc gga ccc gga 366

                60                  65                  70

agg ggc ggt ggc gca ggg tcc tcg cgc gtc cca cgt ggg gtc ccg ggc 414

            75                  80                  85

cca gca gtc tgc gca ccg ggc tcg ctg ctg cac cac gcg tca ccc acg 462


        90                  95                  100

cag acc atg gca gcc gcc gac ggt tcg ctc ttc gac aac ccc agg acg 510

    105                 110                 115

ttc tcc aga cgt ccc cca gcc cag gcg agt cgg caa gca aag gct acg 558

120                 125                 130                 135

aaa aga aaa tac caa gca tcc agt gag gct ccc cca gcg aaa cgg agg 606

                140                 145                 150

aac gaa act tca ttt ctc cca gcc aag aaa act agt gtt aaa gaa act 654

            155                 160                 165

cag agg act ttt aag ggg aac gca caa aaa atg ttt tct cca aag aag 702

        170                 175                 180

cat tcg gtt agc aca agt gat aga aac cag gag gag aga cag tgc att 750

    185                 190                 195

aag act tca tca ctg ttt aaa aac aac cct gac att cca gaa ctc cac 798

200                 205                 210                 215

aga cct gtg gta aag cag gtg caa gaa aaa gtg ttt act tca gct gct 846

                220                 225                 230

ttt cat gag ctg ggc ctc cac cca cat tta att tcc aca ata aat acg 894

            235                 240                 245

gtc tta aaa atg tct agt atg acc agt gtt cag aag caa agt att cct 942

        250                 255                 260

gtg ttg ctg gaa ggc aga gat gct ctc gtg aga tcc cag acg ggc tca 990

    265                 270                 275

ggt aaa act ctt gcc tat tgc atc cct gtg gtc cag tcc ctt caa gca 1038

280                 285                 290                 295

atg gag tca aaa ata cag cgc agt gat ggc ccc tat gcc ctg gtg ctc 1086


300                 305                 310

gtg cca acg aga gag cta gct cta caa agc ttt gac act gtc cag aaa    1134

            315                 320                 325

ctg ctt aag cct ttc acc tgg att gtg cct gga gtg tta atg gga gga    1182

        330                 335                 340

gag aag aga aaa tca gaa aag gcc aga ctc cgc aaa gga ata aat atc    1230

    345                 350                 355

ctt atc tca act cct gga cgc ctg gtg gat cat ata aaa tcc aca aag    1278

360                 365                 370                 375

aac att cat ttt agt cgg ctg cgg tgg ttg gtg ttt gat gaa gca gac    1326

                380                 385                 390

aga atc ttg gat ttg ggt ttt gaa aag gac atc aca gtg ata ctt aat    1374

            395                 400                 405

gct gta aat gct gaa tgc caa aaa cga cag aat gtc ttg cta tca gcg 1422

        410                 415                 420

aca ctc aca gaa ggt gta acg cgg cta gct gat atc agt ttg cat gat 1470

    425                 430                 435

cca gtc agt att tct gtc ctg gac aag agc cat gac cag ttg aac cca 1518

440                 445                 450                 455

aag gac aaa gcg gtc cag gag gtc tgt cct cca cca gct ggc gac aag 1566

                460                 465                 470

ctg gac agc ttt gca ata cca gag agt ctc aag cag cat gtg act gtg 1614

            475                 480                 485

gtt ccc agc aaa ctg agg ctt gtc tgc cta gcg gcc ttc atc ctt cag 1662

        490                 495                 500

aaa tgc aag ttt gag gaa gac cag aag atg gtt gtc ttt ttc tca agt 1710


    505                 510                 515

tgc gag ctg gtg gag ttc cac tac agc ctc ttc cta cag acc ctg ctg 1758

520                 525                 530                 535

agc agc tca ggg gcg ccg gca tca ggg cag ttg cca tct gcc tcc atg 1806

                540                 545                 550

cga tta aaa ttc cta cgg ctg cat ggc ggc atg gag cag gag gaa aga 1854

            555                 560                 565

aca gca gtg ttt cag gaa ttt tca cat tcc aga aga ggc gtc ctt ctt 1902

        570                 575                 580

tgc acg gat gtt gca gct cgg ggc tta gat ctc cct caa gtc acg tgg 1950

    585                 590                 595

att gtt cag tac aac gct cca tct tca cct gca gaa tac atc cac cgg 1998

600                 605                 610                 615

att gga aga acc gcc cgg att ggc tgc cat ggg agc agc ctg ctc att 2046

620 625 630

ttg gct cct tcg gag gca gaa tat gtc aac tcg ttg gct tct cac aaa 2094

635 640 645

atc aac gtt tct gag att aag atg gaa gat att ttg tgt gtt ctg aca 2142

650 655 660

aga gat gat tgt ttt aaa ggg aaa cga tgg gga gcc cag aaa tcc cat 2190

665 670 675

gct gtt ggc ccc cag gaa atc cga gag cga gcc aca gtc ttg cag acg 2238

680 685 690 695

gta ttt gaa gat tac gtg cac tcc agt gag agg agg gtc tcc tgg gca 2286

700 705 710

aag aaa gct ctg cag tcc ttc atc caa gcc tac gcc acc tac ccc agg 2334


715 720 725

gag ctg aag cac atc ttc cac gtc cga tcc ctc cac ctt ggg cat gtg 2382

730 735 740

gcg aag agc ttc gga cta aga gat gcc ccc agg aat ctt agt gcc ttg 2430

745 750 755

act aga aag aag agg aaa gca cac gtg aaa agg cct gac ctt cat aag 2478

760 765 770 775

aag acc cag agt aaa cac agc ctc gct gaa atc cta cgt tcg gaa tac 2526

780 785 790

tca agc ggc atg gag gcc gac gtc gcc aag gtc aaa aag caa aac gca 2574

795 800 805

cct gga gag cct ggt ggc cgg ccc ctg cag cac agt ctg cag ccg aca 2622

810 815 820

ccc tgc ttt ggc cgt ggg aaa aca tta aaa tgg aga aaa acc caa aaa 2670

825 830 835

ggt gta cag cgg gac agc aag act tcc cag aaa gtt taaaatctct 2716

840 845 850

ctgggtcttc gagtggaacc tggaagcccc gggtggcagt ggattgatgt ccagttcttg 2776

tcagaggagc cttgtgaaaa ataactgcgc ctttgcctga ataccacgtc cccaagaatc 2836

agaggggcca cagcccttct cctccatggg aggccctgga gcctgataga aatcagtggt 2896

gtggggcact gtagagacta gccagctccc gacggaaggg gtctgtgtct tgtattgatc 2956

ccatcagtgc ccggcactcc cttcacccag gtgctgacgt gccggggttg agctggcacc 3016

ccgggatgtg atgtaagaac gccctgcgcg tcagcctgga acgggcttga ttgctgccct 3076

taactgtgtt ctgaagtttc acagcttggc ttcagcctat cagcgcatac attggcatga 3136

attttgtgaa ttagggtgtt aaaaactgga attgaatttg tacaaaaaga gaatatattt 3196

atcactaatt attttcttaa tgtaggaatg taccgttaaa aaggaccaaa agtttttggt 3256

ctgggtcaaa aaatacgaat gtgttct                                    3283

<210> 4

<211> 851

<212> PRT

<213> Homo sapiens

<400> 4

<210> 5
<211> 3064
<212> DNA
<213> Homo sapiens

<220>
<221> CDS
<222> (154)..(2487)

<400> 5

aaaaaaggac agcgagcggg gagccccgag gacccccttt tccctgccca gggattgagc 60

tccagagcct gttagggagc tacccagaca ggctttagtt cctaaagtgc tgaggaaacg 120

cacggggcgg taaaggggag gctcgtcggt gag atg gcg ccg gat ctc gct tct 174
Met Ala Pro Asp Leu Ala Ser
1               5

cag agg cat tca gag agc ttc ccc agc gtg aac tct cgg ccg aat gta 222
Gln Arg His Ser Glu Ser Phe Pro Ser Val Asn Ser Arg Pro Asn Val
        10                  15                  20

att ttg cca ggc cgc gag gga cgc aga gag ggg ctg cca cca ggc ggg 270
Ile Leu Pro Gly Arg Glu Gly Arg Arg Glu Gly Leu Pro Pro Gly Gly
    25                  30                  35

ggc acg cgc ggt agt ctt gtc cct acc cgc ccg gtg ccg ccg tcc ccg 318
Gly Thr Arg Gly Ser Leu Val Pro Thr Arg Pro Val Pro Pro Ser Pro
40                  45                  50                  55

gct ccc ctc ggg acg agt ccg tac agc tgg agc cgt tcc gga ccc gga 366
Ala Pro Leu Gly Thr Ser Pro Tyr Ser Trp Ser Arg Ser Gly Pro Gly
                60                  65                  70

agg ggc ggt ggc gca ggg tcc tcg cgc gtc cca cgt ggg gtc ccg ggc 414
Arg Gly Gly Gly Ala Gly Ser Ser Arg Val Pro Arg Gly Val Pro Gly

            75                  80                  85

cca gca gtc tgc gca ccg ggc tcg ctg ctg cac cac gcg tca ccc acg 462
Pro Ala Val Cys Ala Pro Gly Ser Leu Leu His His Ala Ser Pro Thr
        90                  95                  100

cag acc atg gca gcc gcc gac ggt tcg ctc ttc gac aac ccc agg acg 510
Gln Thr Met Ala Ala Ala Asp Gly Ser Leu Phe Asp Asn Pro Arg Thr
    105                 110                 115

ttc tcc aga cgt ccc cca gcc cag gcg agt cgg caa gca aag gct acg 558
Phe Ser Arg Arg Pro Pro Ala Gln Ala Ser Arg Gln Ala Lys Ala Thr
120                 125                 130                 135

aaa aga aaa tac caa gca tcc agt gag gct ccc cca gcg aaa cgg agg 606
Lys Arg Lys Tyr Gln Ala Ser Ser Glu Ala Pro Pro Ala Lys Arg Arg
                140                 145                 150

aac gaa act tca ttt ctc cca gcc aag aaa act agt gtt aaa gaa act 654
Asn Glu Thr Ser Phe Leu Pro Ala Lys Lys Thr Ser Val Lys Glu Thr
            155                 160                 165

cag agg act ttt aag ggg aac gca caa aaa atg ttt tct cca aag aag 702
Gln Arg Thr Phe Lys Gly Asn Ala Gln Lys Met Phe Ser Pro Lys Lys
        170                 175                 180

cat tcg gtt agc aca agt gat aga aac cag gag gag aga cag tgc att 750
His Ser Val Ser Thr Ser Asp Arg Asn Gln Glu Glu Arg Gln Cys Ile
    185                 190                 195
aag act tca tca ctg ttt aaa aac aac cct gac att cca gaa ctc cac 798
Lys Thr Ser Ser Leu Phe Lys Asn Asn Pro Asp Ile Pro Glu Leu His
200                 205                 210                 215
aga cct gtg gta aag cag gtg caa gaa aaa gtg ttt act tca gct gct 846
Arg Pro Val Val Lys Gln Val Gln Glu Lys Val Phe Thr Ser Ala Ala
                220                 225                 230
ttt cat gag ctg ggc ctc cac cca cat tta att tcc aca ata aat acg 894
Phe His Glu Leu Gly Leu His Pro His Leu Ile Ser Thr Ile Asn Thr
            235                 240                 245
gtc tta aaa atg tct agt atg acc agt gtt cag aag caa agt att cct 942
Val Leu Lys Met Ser Ser Met Thr Ser Val Gln Lys Gln Ser Ile Pro
        250                 255                 260
gtg ttg ctg gaa ggc aga gat gct ctc gtg aga tcc cag acg ggc tca 990
Val Leu Leu Glu Gly Arg Asp Ala Leu Val Arg Ser Gln Thr Gly Ser
    265                 270                 275
ggt aaa act ctt gcc tat tgc atc cct gtg gtc cag tcc ctt caa gca 1038
Gly Lys Thr Leu Ala Tyr Cys Ile Pro Val Val Gln Ser Leu Gln Ala

280                 285                 290                 295

atg gag tca aaa ata cag cgc agt gat ggc ccc tat gcc ctg gtg ctc 1086
Met Glu Ser Lys Ile Gln Arg Ser Asp Gly Pro Tyr Ala Leu Val Leu
                300                 305                 310

gtg cca acg aga gag cta gct cta caa agc ttt gac act gtc cag aaa 1134
Val Pro Thr Arg Glu Leu Ala Leu Gln Ser Phe Asp Thr Val Gln Lys
            315                 320                 325

ctg ctt aag cct ttc acc tgg att gtg cct gga gtg tta atg gga gga 1182
Leu Leu Lys Pro Phe Thr Trp Ile Val Pro Gly Val Leu Met Gly Gly
        330                 335                 340

gag aag aga aaa tca gaa aag gcc aga ctc cgc aaa gga ata aat atc 1230
Glu Lys Arg Lys Ser Glu Lys Ala Arg Leu Arg Lys Gly Ile Asn Ile
    345                 350                 355

ctt atc tca act cct gga cgc ctg gtg gat cat ata aaa tcc aca aag 1278
Leu Ile Ser Thr Pro Gly Arg Leu Val Asp His Ile Lys Ser Thr Lys
360                 365                 370                 375

aac att cat ttt agt cgg ctg cgg tgg ttg gtg ttt gat gaa gca gac 1326
Asn Ile His Phe Ser Arg Leu Arg Trp Leu Val Phe Asp Glu Ala Asp
                380                 385                 390

aga atc ttg gat ttg ggt ttt gaa aag gac atc aca gtg ata ctt aat 1374
Arg Ile Leu Asp Leu Gly Phe Glu Lys Asp Ile Thr Val Ile Leu Asn
            395                 400                 405

gct gta aat gct gaa tgc caa aaa cga cag aat gtc ttg cta tca gcg 1422
Ala Val Asn Ala Glu Cys Gln Lys Arg Gln Asn Val Leu Leu Ser Ala
        410                 415                 420

aca ctc aca gaa ggt gta acg cgg cta gct gat atc agt ttg cat gat 1470
Thr Leu Thr Glu Gly Val Thr Arg Leu Ala Asp Ile Ser Leu His Asp
    425                 430                 435

cca gtc agt att tct gtc ctg gac aag agc cat gac cag ttg aac cca 1518
Pro Val Ser Ile Ser Val Leu Asp Lys Ser His Asp Gln Leu Asn Pro
440                 445                 450                 455

aag gac aaa gcg gtc cag gag gtc tgt cct cca cca gct ggc gac aag 1566
Lys Asp Lys Ala Val Gln Glu Val Cys Pro Pro Pro Ala Gly Asp Lys
                460                 465                 470

ctg gac agc ttt gca ata cca gag agt ctc aag cag cat gtg act gtg 1614
Leu Asp Ser Phe Ala Ile Pro Glu Ser Leu Lys Gln His Val Thr Val
            475                 480                 485

gtt ccc agc aaa ctg agg ctt gtc tgc cta gcg gcc ttc atc ctt cag 1662
Val Pro Ser Lys Leu Arg Leu Val Cys Leu Ala Ala Phe Ile Leu Gln

490                 495                 500

aaa tgc aag ttt gag gaa gac cag aag atg gtt gtc ttt ttc tca agt     1710
Lys Cys Lys Phe Glu Glu Asp Gln Lys Met Val Val Phe Phe Ser Ser
    505                 510                 515

tgc gag ctg gtg gag ttc cac tac agc ctc ttc cta cag acc ctg ctg     1758
Cys Glu Leu Val Glu Phe His Tyr Ser Leu Phe Leu Gln Thr Leu Leu
520                 525                 530                 535

agc agc tca ggg gcg ccg gca tca ggg cag ttg cca tct gcc tcc atg     1806
Ser Ser Ser Gly Ala Pro Ala Ser Gly Gln Leu Pro Ser Ala Ser Met
                540                 545                 550

cga tta aaa ttc cta cgg ctg cat ggc ggc atg gag cag gag gaa aga     1854
Arg Leu Lys Phe Leu Arg Leu His Gly Gly Met Glu Gln Glu Glu Arg
            555                 560                 565

aca gca gtg ttt cag gaa ttt tca cat tcc aga aga ggc gtc ctt ctt     1902
Thr Ala Val Phe Gln Glu Phe Ser His Ser Arg Arg Gly Val Leu Leu
        570                 575                 580

tgc acg gat gtt gca gct cgg ggc tta gat ctc cct caa gtc acg tgg     1950
Cys Thr Asp Val Ala Ala Arg Gly Leu Asp Leu Pro Gln Val Thr Trp
    585                 590                 595

att gtt cag aat aca tcc acc gga ttg gaa gaa ccg ccc gga ttg gct 1998
Ile Val Gln Asn Thr Ser Thr Gly Leu Glu Glu Pro Pro Gly Leu Ala
600                 605                 610                 615

gcc atg gga gca gcc tgc tca ttt tgg ctc ctt cgg agg cag aat atg 2046
Ala Met Gly Ala Ala Cys Ser Phe Trp Leu Leu Arg Arg Gln Asn Met
                620                 625                 630

tca act cgt tgg ctt ctc aca aaa tca act ctg cag tcc ttc atc caa 2094
Ser Thr Arg Trp Leu Leu Thr Lys Ser Thr Leu Gln Ser Phe Ile Gln
            635                 640                 645

gcc tac gcc acc tac ccc agg gag ctg aag cac atc ttc cac gtc cga 2142
Ala Tyr Ala Thr Tyr Pro Arg Glu Leu Lys His Ile Phe His Val Arg
        650                 655                 660

tcc ctc cac ctt ggg cat gtg gcg aag agc ttc gga cta aga gat gcc 2190
Ser Leu His Leu Gly His Val Ala Lys Ser Phe Gly Leu Arg Asp Ala
    665                 670                 675

ccc agg aat ctt agt gcc ttg act aga aag aag agg aaa gca cac gtg 2238
Pro Arg Asn Leu Ser Ala Leu Thr Arg Lys Lys Arg Lys Ala His Val
680                 685                 690                 695

aaa agg cct gac ctt cat aag aag acc cag agt aaa cac agc ctc gct 2286
Lys Arg Pro Asp Leu His Lys Lys Thr Gln Ser Lys His Ser Leu Ala

                700                 705                 710

gaa atc cta cgt tcg gaa tac tca agc ggc atg gag gcc gac gtc gcc 2334
Glu Ile Leu Arg Ser Glu Tyr Ser Ser Gly Met Glu Ala Asp Val Ala
            715                 720                 725

aag gtc aaa aag caa aac gca cct gga gag cct ggt ggc cgg ccc ctg 2382
Lys Val Lys Lys Gln Asn Ala Pro Gly Glu Pro Gly Gly Arg Pro Leu
        730                 735                 740

cag cac agt ctg cag ccg aca ccc tgc ttt ggc cgt ggg aaa aca tta 2430
Gln His Ser Leu Gln Pro Thr Pro Cys Phe Gly Arg Gly Lys Thr Leu
    745                 750                 755

aaa tgg aga aaa acc caa aaa ggt gta cag cgg gac agc aag act tcc 2478
Lys Trp Arg Lys Thr Gln Lys Gly Val Gln Arg Asp Ser Lys Thr Ser
760                 765                 770                 775

cag aaa gtt taaaatctct ctgggtcttc gagtggaacc tggaagcccc 2527
Gln Lys Val

gggtggcagt ggattgatgt ccagttcttg tcagaggagc cttgtgaaaa ataactgcgc 2587

ctttgcctga ataccacgtc cccaagaatc agaggggcca cagcccttct cctccatggg 2647

aggccctgga gcctgataga aatcagtggt gtggggcact gtagagacta gccagctccc 2707

gacggaaggg gtctgtgtct tgtattgatc ccatcagtgc ccggcactcc cttcacccag 2767

gtgctgacgt gccggggttg agctggcacc ccgggatgtg atgtaagaac gccctgcgcg 2827

tcagcctgga acgggcttga ttgctgccct taactgtgtt ctgaagtttc acagcttggc 2887

ttcagcctat cagcgcatac attggcatga attttgtgaa ttagggtgtt aaaaactgga 2947

attgaatttg tacaaaaaga gaatatattt atcactaatt attttcttaa tgtaggaatg 3007

taccgttaaa aaggaccaaa agtttttggt ctgggtcaaa aaatacgaat gtgttct 3064

<210> 6

<211> 778

<212> PRT

<213> Homo sapiens

<400> 6
   Met Ala Pro Asp Leu Ala Ser Gln Arg His Ser Glu Ser Phe Pro Ser
   1 5 10 15
   Val Asn Ser Arg Pro Asn Val Ile Leu Pro Gly Arg Glu Gly Arg Arg
   20 25 30
   Glu Gly Leu Pro Pro Gly Gly Gly Thr Arg Gly Ser Leu Val Pro Thr
   35 40 45
   Arg Pro Val Pro Pro Ser Pro Ala Pro Leu Gly Thr Ser Pro Tyr Ser
   50 55 60
   Trp Ser Arg Ser Gly Pro Gly Arg Gly Gly Gly Ala Gly Ser Ser Arg
   65 70 75 80
   Val Pro Arg Gly Val Pro Gly Pro Ala Val Cys Ala Pro Gly Ser Leu
   85 90 95
   Leu His His Ala Ser Pro Thr Gln Thr Met Ala Ala Ala Asp Gly Ser
   100 105 110
   Leu Phe Asp Asn Pro Arg Thr Phe Ser Arg Arg Pro Pro Ala Gln Ala
   115 120 125
   Ser Arg Gln Ala Lys Ala Thr Lys Arg Lys Tyr Gln Ala Ser Ser Glu
   130 135 140
   Ala Pro Pro Ala Lys Arg Arg Asn Glu Thr Ser Phe Leu Pro Ala Lys
   145 150 155 160
   Lys Thr Ser Val Lys Glu Thr Gln Arg Thr Phe Lys Gly Asn Ala Gln
   165 170 175
   Lys Met Phe Ser Pro Lys Lys His Ser Val Ser Thr Ser Asp Arg Asn
   180 185 190
   Gln Glu Glu Arg Gln Cys Ile Lys Thr Ser Ser Leu Phe Lys Asn Asn
   195 200 205
   Pro Asp Ile Pro Glu Leu His Arg Pro Val Val Lys Gln Val Gln Glu
   210 215 220
   Lys Val Phe Thr Ser Ala Ala Phe His Glu Leu Gly Leu His Pro His
   225 230 235 240
   Leu Ile Ser Thr Ile Asn Thr Val Leu Lys Met Ser Ser Met Thr Ser
   245 250 255
   Val Gln Lys Gln Ser Ile Pro Val Leu Leu Glu Gly Arg Asp Ala Leu
   260 265 270
   Val Arg Ser Gln Thr Gly Ser Gly Lys Thr Leu Ala Tyr Cys Ile Pro
   275 280 285
   Val Val Gln Ser Leu Gln Ala Met Glu Ser Lys Ile Gln Arg Ser Asp
   290 295 300
   Gly Pro Tyr Ala Leu Val Leu Val Pro Thr Arg Glu Leu Ala Leu Gln
   305 310 315 320
   Ser Phe Asp Thr Val Gln Lys Leu Leu Lys Pro Phe Thr Trp Ile Val
   325 330 335
   Pro Gly Val Leu Met Gly Gly Glu Lys Arg Lys Ser Glu Lys Ala Arg
   340 345 350
   Leu Arg Lys Gly Ile Asn Ile Leu Ile Ser Thr Pro Gly Arg Leu Val
   355 360 365
   Asp His Ile Lys Ser Thr Lys Asn Ile His Phe Ser Arg Leu Arg Trp
   370 375 380
   Leu Val Phe Asp Glu Ala Asp Arg Ile Leu Asp Leu Gly Phe Glu Lys
   385 390 395 400
   Asp Ile Thr Val Ile Leu Asn Ala Val Asn Ala Glu Cys Gln Lys Arg
   405 410 415
   Gln Asn Val Leu Leu Ser Ala Thr Leu Thr Glu Gly Val Thr Arg Leu
   420 425 430
   Ala Asp Ile Ser Leu His Asp Pro Val Ser Ile Ser Val Leu Asp Lys
   435 440 445
   Ser His Asp Gln Leu Asn Pro Lys Asp Lys Ala Val Gln Glu Val Cys
   450 455 460
   Pro Pro Pro Ala Gly Asp Lys Leu Asp Ser Phe Ala Ile Pro Glu Ser
   465 470 475 480
   Leu Lys Gln His Val Thr Val Val Pro Ser Lys Leu Arg Leu Val Cys
   485 490 495
   Leu Ala Ala Phe Ile Leu Gln Lys Cys Lys Phe Glu Glu Asp Gln Lys
   500 505 510
   Met Val Val Phe Phe Ser Ser Cys Glu Leu Val Glu Phe His Tyr Ser
   515 520 525
   Leu Phe Leu Gln Thr Leu Leu Ser Ser Ser Gly Ala Pro Ala Ser Gly
   530 535 540
   Gln Leu Pro Ser Ala Ser Met Arg Leu Lys Phe Leu Arg Leu His Gly
   545 550 555 560
   Gly Met Glu Gln Glu Glu Arg Thr Ala Val Phe Gln Glu Phe Ser His
   565 570 575
   Ser Arg Arg Gly Val Leu Leu Cys Thr Asp Val Ala Ala Arg Gly Leu
   580 585 590
   Asp Leu Pro Gln Val Thr Trp Ile Val Gln Asn Thr Ser Thr Gly Leu
   595 600 605
   Glu Glu Pro Pro Gly Leu Ala Ala Met Gly Ala Ala Cys Ser Phe Trp
   610 615 620
   Leu Leu Arg Arg Gln Asn Met Ser Thr Arg Trp Leu Leu Thr Lys Ser
   625 630 635 640
   Thr Leu Gln Ser Phe Ile Gln Ala Tyr Ala Thr Tyr Pro Arg Glu Leu
   645 650 655
   Lys His Ile Phe His Val Arg Ser Leu His Leu Gly His Val Ala Lys
   660 665 670
   Ser Phe Gly Leu Arg Asp Ala Pro Arg Asn Leu Ser Ala Leu Thr Arg
   675 680 685
   Lys Lys Arg Lys Ala His Val Lys Arg Pro Asp Leu His Lys Lys Thr
   690 695 700
   Gln Ser Lys His Ser Leu Ala Glu Ile Leu Arg Ser Glu Tyr Ser Ser
   705 710 715 720
   Gly Met Glu Ala Asp Val Ala Lys Val Lys Lys Gln Asn Ala Pro Gly
   725 730 735
   Glu Pro Gly Gly Arg Pro Leu Gln His Ser Leu Gln Pro Thr Pro Cys
   740 745 750
   Phe Gly Arg Gly Lys Thr Leu Lys Trp Arg Lys Thr Gln Lys Gly Val
   755 760 765
   Gln Arg Asp Ser Lys Thr Ser Gln Lys Val
   770 775

<210> 7

<211> 21

<212> DNA

<213> Artificial Sequence

<220>

<223> Description of Artificial Sequence: Artificially

      Synthesized Primer Sequence

<400> 7

ttcattgacc tcaactacat g                                                21

<210> 8

<211> 20

<212> DNA

<213> Artificial Sequence

<220>

<223> Description of Artificial Sequence: Artificially

Synthesized Primer Sequence

<400> 8

gtggcagtga tggcatggac 20

<210> 9

<211> 23

<212> DNA

<213> Artificial Sequence

<220>

<223> Description of Artificial Sequence: Artificially

Synthesized Primer Sequence

<400> 9

aataccaagc atccagtgag gct 23

<210> 10

<211> 20

<212> DNA

<213> Artificial Sequence

<220>

<223> Description of Artificial Sequence: Artificially

Synthesized Primer Sequence

<400> 10

gcaataggca agagttttac                                                    20

<210> 11

<211> 20

<212> DNA

<213> Artificial Sequence

<220>

<223> Description of Artificial Sequence: Artificially

Synthesized Primer Sequence

<400> 11

ctggtcatac tagacatttt                                                    20

<210> 12

<211> 27

<212> DNA

<213> Artificial Sequence

<220>

<223> Description of Artificial Sequence: Artificially

Synthesized Primer Sequence

<400> 12

ccatcctaat actactcact atagggc 27

<210> 13

<211> 20

<212> DNA

<213> Artificial Sequence

<220>

<223> Description of Artificial Sequence: Artificially

Synthesized Primer Sequence

<400> 13

agtcctctga gtttctttaa 20

<210> 14

<211> 23

<212> DNA

<213> Artificial Sequence

<220>

<223> Description of Artificial Sequence: Artificially

Synthesized Primer Sequence

<400> 14

actcactata gggctcgagc ggc 23

<210> 15

<211> 21

<212> DNA

<213> Artificial Sequence

<220>

<223> Description of Artificial Sequence: Artificially

Synthesized Primer Sequence

<400> 15

accctaattc acaaaattca t 21

<210> 16

<211> 21

<212> DNA

<213> Artificial Sequence

<220>

<223> Description of Artificial Sequence: Artificially

Synthesized Primer Sequence

<400> 16

aactttctgg gaagtcttgc t 21

<210> 17

<211> 30

<212> DNA

<213> Artificial Sequence

<220>

<223> Description of Artificial Sequence: Artificially

Synthesized Primer Sequence

<400> 17

ggatccaaga tggcgccgga tctcgcttct 30

<210> 18

<211> 21

<212> DNA

<213> Artificial Sequence

<220>

<223> Description of Artificial Sequence: Artificially

Synthesized Primer Sequence

<400> 18

aactttctgg gaagtcttgc t 21

<210> 19

<211> 21

<212> DNA

<213> Artificial Sequence

<220>

<223> Description of Artificial Sequence: Artificially

Synthesized Primer Sequence

<400> 19

cttggtctct gcagccaaac a 21

<210> 20

<211> 22

<212> DNA

<213> Artificial Sequence

<220>

<223> Description of Artificial Sequence: Artificially

Synthesized Primer Sequence

<400> 20

gaactttctg ggaagtcttg ct 22

## Description

### Technical Field

The present invention belongs to the field of bioscience, and specifically, relates to transcription factors. More specifically, the present invention relates to novel RNA helicases and genes encoding the helicases. These molecules can be used, for example, in the field of developing pharmaceuticals.

### Background Art

Transcription of genes, essential for the initial steps in gene expression, is a process comprising several steps and is related to many events that are essential *in vivo.* Transcription by RNA polymerase is a mechanism that is commonly seen in transcription with all transcriptional regulating factors. Three types of RNA polymerases have been known in the art: (1) RNA polymerase I, which synthesizes ribosomal RNA (hereinafter, abbreviated as rRNA) in the nucleolus; (2) RNA polymerase II, which synthesizes mRNA; and (3) RNA polymerase III, which is involved in the synthesis of transfer RNA (hereinafter, abbreviated as tRNA). Recently, RNA helicases have been revealed to form a complex with RNA polymerase or other transcription factors (BRCA1, estrogen receptor, etc.) to control the function of RNA polymerase (Cell Technology (SAIBOU KOUGAKU), Vol. 17 No. 10, 1998, 1512-1516, "The role of RNA helicase A complex in gene expression", Takayuki Ohshima, Akiyoshi Fukamizu, Toshihiro Nakajima; Anderson S.F., et al., Nature Genet. (1998) 19: 254-256; Endoh, H. et al, 1999, Mol. Cell. Biol. 19: 5363-72). RNA helicase belongs to the helicase family, and comprise a DEAD/H box structure in their amino acid sequence. Some of the RNA helicases have been reported to have an enzyme activity to dissociate RNA complexes in cells and an enzyme activity as ATPase. Though only such RNA helicases that regulate transcription through the interaction with RNA polymerase II have been known in the art, RNA helicase may be involved in rRNA synthesis mediated by RNA polymerase I. However, the interaction between rRNA synthesis or polymerase and RNA helicase has not been investigated at all. Moreover, though rRNA synthesis has been suggested to be involved in cell proliferation, the mechanisms of upregulation of rRNA synthesis in cancer cells has remained obscure.

### Disclosure of the Invention

The object of the present invention is to provide novel RNA helicase proteins, and genes encoding them, as well as methods for producing and using the same.

In order to isolate genes encoding proteins interacting with testis specific protein, Tesmin, the present inventors conducted yeast Two-Hybrid method to discover novel gene sequences. The present inventors analyzed the isolated genes, Helicains, which were revealed to consist of novel gene sequences demonstrating no significant homology to other genes on databases. Moreover, analyzing the proteins encoded by the genes, the present inventors discovered that the proteins included a partial structure similar to RNA helicase which is known as a member of the helicase family. Further, the sequences of Helicain B protein and Helicain C protein included a DEAD box structure and GxGKx, which are sequence structures common among RNA helicases. Accordingly, the "Helicain" proteins may function as RNA helicases to regulate transcription, and so on. Moreover, as a result of expression analysis in tissues, the genes were revealed to be expressed significantly higher in multiple tumor cells and normal thyroid tissue compared with normal tissues. Furthermore, analyzing the position of the gene on human chromosomes, the genes were demonstrated to be located at a similar locus as that reported to be abnormal in multiple cancer cells. According to these results, the "Helicains" may be applicable as novel cancer cell markers by detecting their expression level in cancer cells. Moreover, the "Helicain" proteins of the present invention will be useful as tools for elucidating the mechanism of cancer and as targets for developing pharmaceuticals for cancer.

The present invention relates to novel RNA helicase proteins, and genes encoding the proteins, as well as methods for producing and using the same. More specifically, the present invention relates to:
(1) a DNA selected from the group consisting of:
   (a) a DNA encoding a protein comprising the amino acid sequence of SEQ ID NO: 2, 4 or 6; and
   (b) a DNA comprising the coding region of the nucleotide sequence of SEQ ID NO: 1, 3 or 5;
(2) a DNA encoding a protein having the activity to bind with Tesmin protein selected from the group consisting of:
   (a) a DNA encoding a protein comprising the amino acid sequence of SEQ ID NO: 2, 4 or 6 in which one or more amino acids are substituted, deleted, inserted, and/or added; and
   (b) a DNA hybridizing with a DNA consisting of the nucleotide sequence of SEQ ID NO: 1, 3 or 5;
(3) a DNA encoding a protein having RNA helicase activity selected from the group consisting of:
   (a) a DNA encoding a protein comprising the amino acid sequence of SEQ ID NO: 4 or 6 in which one or more amino acids are substituted, deleted, inserted, and/or added; and
   (b) a DNA hybridizing with a DNA consisting of the nucleotide sequence of SEQ ID NO: 3 or 5;
(4) a protein encoded by the DNA of any one of (1) to (3);
(5) a vector into which the DNA of any one of (1) to (3) is inserted;
(6) a host cell retaining the vector of (5);
(7) a method for producing the protein of (4), comprising the steps of: culturing the host cell of (6), and recovering the expressed protein from said host cell or the culture supernatant thereof;
(8) a partial peptide of the protein of (4);
(9) a polynucleotide complementary to either a DNA that comprises the nucleotide sequence of SEQ ID NO: 1, 3 or 5, or its complementary strand, wherein the polynucleotide comprises at least 15 nucleotides;
(10) a method of screening for a compound that binds to the protein of (4), comprising the steps of:
   (a) contacting the protein or partial peptide thereof with a test sample;
   (b) detecting the binding activity of the protein or partial peptide thereof with the test sample; and
   (c) selecting the compound that binds to the protein or partial peptide thereof;
(11) an antibody binding to the protein of (4);
(12) a compound binding to the protein of (4), which is isolated by the method of (10);
(13) a method of examining for cancer, comprising the step of detecting mRNA that encodes the protein of (4) in the test sample;
(14) a method of examining for cancer, comprising the step of detecting the protein of (4) in the test sample;
(15) a reagent for examining for cancer, comprising the antibody of (11);
(16) a method of screening for a compound that regulates the binding between the protein of (4) and Tesmin, comprising the steps of:
   (a) contacting the protein of (4) with Tesmin in the presence of a test sample;
   (b) detecting the binding between the protein of (4) and Tesmin; and
   (c) selecting the compound that suppresses or promotes the binding compared with that observed in the absence of the test sample;
(17) a compound regulating the binding between the protein of (4) and Tesmin, which is isolated by the method of (16) ;
(18) a method of screening for a compound that regulates RNA helicase activity of a protein comprising the amino acid sequence of SEQ ID NO: 4 or 6, or a protein encoded by the DNA of (3), comprising the steps of:
   (a) contacting a double stranded nucleic acid as a substrate to the protein in the presence of a test sample;
   (b) detecting the dissociation of single strand RNA from the double stranded nucleic acid; and
   (c) selecting the compound that suppresses or promotes the dissociation compared with that observed in the absence of the test sample;
(19) a compound regulating RNA helicase activity of a protein comprising the amino acid sequence of SEQ ID NO: 4 or 6, or a protein encoded by the DNA of (3), wherein the compound is isolated by the method of (18);
(20) a method of screening for a compound that regulates the localization into the nucleolus of the protein of (4), comprising the steps of:
   (a) contacting a test sample with or introducing a test sample into cells expressing the protein;
   (b) detecting the cellular localization of the protein; and
   (c) selecting the compound that suppresses or promotes the localization of the protein into the nucleolus compared with that observed in the absence of the test sample;
(21) a compound regulating the nuclear localization of the protein of (4), wherein the compound is isolated by the method of (20);
(22) a pharmaceutical composition comprising the compound of (12), (17), (19) or (21) as the active ingredient; and
(23) an anticancer agent comprising, as the active ingredient, a compound that suppresses RNA helicase activity of a protein comprising the aminoacid sequence of SEQ ID NO: 4 or 6, or the protein encoded by the DNA of (3), which compound is isolated by the method of (18); or a compound suppressing the localization into nucleolus of the protein of (4), which compound is isolated by the method of (20).

The present invention relates to genes "Helicains" encoding novel proteins that have RNA helicase structure. The nucleotide sequences of human Helicain A, B, and C cDNA are shown in SEQ ID NO: 1, 3, and 5, respectively, and the amino acid sequences of the proteins encoded by the cDNAs are shown in SEQ ID NO: 2, 4, and 6, respectively. As shown in SEQ ID NO: 1, the human Helicain A cDNA comprises an ORF encoding a protein consisting of 319 amino acids. As shown in SEQ ID NO: 3, the human Helicain B cDNA comprises an ORF encoding a protein consisting of 851 amino acids. As shown in SEQ ID NO: 5, the human Helicain C cDNA comprises an ORF encoding a protein consisting of 778 amino acids.

Further, human "Helicain" genes were demonstrated to be strongly expressed in thyroid gland and weakly in other tissues of the body by Northern hybridization analysis and RT-PCR analysis. The proteins of the present invention are useful as novel markers for cancer cells, and serve also as targets for developing pharmaceuticals for cancer.

The present invention also includes proteins that are functionally equivalent to the human "Helicain" proteins (SEQ ID NO: 2, 4, and 6). Such proteins include, for example, proteins structurally similar to the human "Helicain" proteins (SEQ ID NO: 2, 4, and 6) that bind with Tesmin protein (Sugihara, T. et al., 1999, Genomics 57: 130-136). Such activity can be detected, for example, by yeast two-hybrid system. Alternatively, the activity can be also measured, for example, by the pull-down assay, measurement utilizing surface plasmon resonance, ELISA, and so on. The proteins of the present invention may also bind to other transcription factors.

Proteins functionally equivalent to the human "Helicain" proteins of the present invention (SEQ ID NO: 4 and 6) also encompass such proteins that are structurally similar to the human "Helicain" proteins of the present invention (SEQ ID NO: 4 and 6) having RNA helicase activity. The term "RNA helicase activity" refers to the activity to unwind double stranded nucleic acids, which comprise RNA, to single strands. More specifically, assay method of RNA helicase activity includes such methods as follows.

First, single stranded RNA is fixed on a plate, a Digoxigenin-labeled RNA complementary thereto is bound to the fixed RNA to prepare a double-stranded RNA. The sequence of the RNA strand is not limited in any way. However, RNAs with a length of around 40 bp is desirable. A test protein is added thereto, and the amount of labeled RNA strand on the plate is measured by the ELISA method to determine the decrease of the amount as the activity (Hsu, C.C. et al., 1998, Biochem. Biophys Res. Commun. 253: 594-599). Generally, methods using radioisotopes as the label to measure the activity with PAGE gels are frequently used (Tai, C.L. et al., 1996, J. Virol. 70: 8477-8484).

Whether a protein has the RNA helicase activity or not can be determined by the existence of a GxGKx sequence (G: glycine, K: lysine, x: arbitrary amino acid), and DEAD/H motif (D: aspartic acid, E: glutamic acid, A: alanine, D/H: aspartic acid or histidine) in its amino acid sequence. The DEAD/H motif is required for Mg²⁺ chelation, and the GxGKx sequence is considered as the NTP binding sequence (Kanai, A. et al., 1995, FEBS Lett. 376: 221-224; Schmid, S.R. and Linder, P., 1992, Mol. Microbiol. 6: 283-291). Generally, proteins having the GxGKx sequence and DEAD/H motif in their amino acid sequences are considered to possess RNA helicase activity (Yao, N. et al., Nat. Struct. Biol. 4: 463-467 (1997); Kim, J.L. et al., Structure 6: 89-100 (1998); Luking, A. et al., Crit. Rev. Biochem. Mol. Biol. 33(4): 259-96 (1998)).

Proteins functionally equivalent to the human "Helicain" proteins (SEQ ID NO: 2, 4, and 6) as described above include, for example, mutants, alleles, variants, homologs, and such, of human "Helicain" proteins.

Methods for introducing mutations for preparing proteins that are functionally equivalent to other proteins are well known to those skilled in the art. For example, one may use site-directed mutagenesis (Hashimoto-Goto, T. et al., Gene 152: 271-275 (1995); Zoller, M.J. and Smith M., Methods Enzymol. 100: 468-500 (1983); Kramer, W. et al., Nucleic Acids Res. 12: 9441-9456 (1984); Kramer, W. and Fritz H.J., Methods. Enzymol. 154: 350-367 (1987); Kunkel, T.A., Proc. Natl. Acad. Sci. U.S.A. 82: 488-492 (1985); Kunkel, Methods Enzymol. 85: 2763-2766 (1988)), and such in order to introduce an appropriate mutation into the amino acid sequence of a human Helicain protein (SEQ ID NO: 2, 4, or 6) to prepare proteins that are functionally equivalent to the protein. Mutations of amino acids may occur in nature as well. This invention includes proteins having the amino acid sequence of a human Helicain protein (SEQ ID NO: 2, 4, or 6) in which one or more amino acid residues are mutated, and wherein the proteins are functionally equivalent to the protein. In such a mutant protein, the number of the amino acids to be mutated is thought to be usually 100 residues or less, preferably 60 residues or less, and more preferably 30 residues or less.

As for the amino acid residue to be mutated, it is preferable that it be mutated into a different amino acid that allows the properties of the amino acid side-chain are conserved. Examples of properties of amino acid side chains are the following: hydrophobic amino acids (A, I, L, M, F, P, W, Y, V), hydrophilic amino acids (R, D, N, C, E, Q, G, H, K, S, T), and amino acids comprising the following side chains: an aliphatic side-chain (G, A, V, L, I, P); a hydroxyl group containing side-chain(S, T, Y); a sulfur atom containing side-chain (C, M); a carboxylic acid and amide containing side-chain (D, N, E, Q); a base-containing side-chain (R, K, H); and an aromatic-containing side-chain (H, F, Y, W) (The parenthetic letters indicate the one-letter codes of amino acids).

It is known that a protein having an amino acid sequence which is modified by deletion, addition, and/or substitution by other amino acids of one or more amino acid residues, yet still retain its biological activity (Mark, D. F. et al., Proc. Natl. Acad. Sci. USA 81: 5662-5666 (1984); Zoller, M. J. & Smith, M., Nucleic Acids Research 10: 6487-6500 (1982); Wang, A. et al., Science 224: 1431-1433; Dalbadie-McFarland, G. et al., Proc. Natl. Acad. Sci. USA79: 6409-6413(1982)).

A fusion protein containing a human Helicain protein is an example of a protein in which two or more amino acid residues have been added to the amino acid sequence of the human Helicain protein. A fusion protein is made by fusing a human Helicain protein with another peptide(s) or protein(s) and is included in the present invention. A fusion protein can be prepared by ligating a DNA encoding a human Helicain protein (SEQ ID NO: 2, 4, or 6) with a DNA encoding another peptide(s) or protein(s) in frame, introducing the ligated DNA into an expression vector, and expressing the fused gene in a host. Methods known by those skilled in the art can be used for preparing such a fusion gene. There is no restriction as to the other peptide(s) or protein(s) that is (are) fused to the protein of this invention.

Other peptide(s) to be fused with a protein of the present invention are known peptides including, for example, FLAG (Hopp, T.P. et al., Biotechnology 6: 1204-1210 (1988)), 6x His constituting six histidine (His) residues, 10x His, Influenza agglutinin (HA), human c-myc fragment, VSV-GP fragment, p18HIV fragment, T7-tag, HSV-tag, E-tag, SV40T antigen fragment, lck tag, α-tubulin fragment, B-tag, Protein C fragment, and so on. Other examples of peptides to be fused with the protein of the present invention include glutathione-S-transferase (GST), Influenza agglutinin (HA), immunoglobulin constant region, β-galactosidase, maltose-binding protein (MBP), etc. Fusion proteins can be prepared by fusing commercially available DNA encoding these peptides or proteins with DNA encoding a protein of the present invention and expressing the fused DNA prepared.

The hybridization technique (Sambrook, J et al., Molecular Cloning 2nd ed. 9.47-9.58, Cold Spring Harbor Lab. press, (1989)) is well known to those skilled in the art as an alternative method for preparing proteins functionally equivalent to a certain protein. Specifically, those skilled in the art can ordinarily isolate highly homologous DNAs based on the DNA sequence (SEQ ID NO: 1, 3, or 5) encoding a human "Helicain" protein or partial sequence thereof, and prepare proteins functionally equivalent to the human "Helicain" proteins from the DNAs. The present invention includes proteins functionally equivalent to the human "Helicain" proteins encoded by DNAs hybridizing with a DNA encoding the human "Helicain" proteins. Such proteins include, for example, non-human mammalian homologs (for example, proteins encoded by mice, rats, rabbits, bovines, and so on). In the case of isolating cDNAs highly homologous to the DNAs encoding the human "Helicain" proteins, many kinds of tissues and cells, such as thyroid gland, testis, and so on, that are expected to express a protein of the present invention can be used.

The condition of hybridization to isolate DNAs encoding proteins functionally equivalent to the human "Helicain" proteins can be properly selected by those skilled in the art. For example, after prehybridization at 68°C for 30 min or longer using "Rapid-hyb buffer" (Amersham LIFE SCIENCE), labeled probe is added, and hybridization is conducted by incubating at 68°C for 1 hr or longer. Followed by washing three times for 20 min at room temperature in 2x SSC and 0.01% SDS; three times for 20 min at 37°C in 1x SSC and 0.1% SDS; and two times for 20 min at 50°C in 1x SSC and 0.1% SDS. However, several factors, such as temperature, and salt concentration, can influence the stringency of hybridization and one skilled in the art can suitably select the factors to accomplish a similar stringency.

In place of hybridization, gene amplification methods using primers synthesized based on the sequence information of the DNAs (SEQ ID NO: 1, 3, and 5) encoding the human "Helicain" proteins, for example, the polymerase chain reaction (PCR) method, can be utilized for the isolation.

A protein functionally equivalent to a human "Helicain" protein encoded by a DNA isolated through the above hybridization techniques or gene amplification techniques normally have a high homology to the human "Helicain" protein (SEQ ID NO: 2, 4, or 6) at the amino acid level. The proteins of the present invention also include proteins that have a high homology to the amino acid sequence shown in SEQ ID NO: 2, 4, or 6, so long as the proteins are functionally equivalent to the human "Helicain" proteins. "High homology" refers to, normally an identity of at least 25% or higher, preferably 40% or higher, more preferably 60% or higher, and more preferably 80% or higher at the amino acid level.

The identity of two amino acid sequences or of two nucleotide sequences is determined using the BLAST algorithm of Karlin and Altschul (Proc. Natl. Acad. Sei. USA 90:5873-5877, 1993). Such an algorithm is incorporated into the BLASTN and BLASTX programs of Altschul et al. (J. Mol. Biol.215:403-410, 1990). BLAST nucleotide searches are performed with the BLASTN program, score = 100, wordlength = 12. BLAST protein searches are performed with the BLASTX program, score = 50, wordlength = 3. When utilizing BLAST and Gapped BLAST programs, the default parameters of the respective programs are used. Specific procedures of these analyses are well known in the art (http://www.ncbi.nlm.nih.gov.).

The amino acid sequence, molecular weight, isoelectric point, the presence or absence of sugar chains, and the form of a protein of the present invention may differ according to the producing cells, host, or purification method described below. However, so long as the obtained protein has an equivalent function to the human Helicain proteins, it is included in the present invention. For example, if a protein of the present invention is expressed in prokaryotic cells, such as *E. coli,* a methionine residue is added at the N-terminus of the amino acid sequence of the expressed protein. Such proteins are also included in the proteins of the present invention.

A protein of the present invention can be prepared by methods known to those skilled in the art, as a recombinant protein, and also as a natural protein. A recombinant protein can be prepared by inserting a DNA encoding a protein of the present invention (for example, the DNA comprising the nucleotide sequence of SEQ ID NO: 1, 3 or 5) into a suitable expression vector, introducing the vector into a suitable host cell, and collecting the resulting transformant. After obtaining the extract from the transformant, recombinant protein can be purified and prepared by subjecting the obtained extract to chromatography, such as ion exchange chromatography, reverse phase chromatography, and gel filtration; affinity chromatography, to which antibodies against a protein of the invention are immobilized; or combination of one or more of these columns.

Further, when a protein of the present invention is expressed within host cells (for example, animal cells and *E. coli*), as a fusion protein with glutathione-S-transferase protein or as a recombinant protein supplemented with multiple histidines, the expressed recombinant protein can be purified using a glutathione column or nickel column. After purifying the fusion protein, it is also possible to exclude regions other than the objective protein by cutting with thrombin, factor-Xa, and such, as required.

A natural protein may be isolated by methods known to those skilled in the art. For example, to isolate the natural protein, extracts of tissue or cells expressing a protein of the invention may be reacted with an affinity column described below, to which antibodies binding to the protein of the invention are attached. The antibodies may be polyclonal or monoclonal antibodies.

This invention also includes partial peptides of the proteins of the invention. The peptide of the invention are composed of at least 7 amino acids or more, preferably 8 amino acids or more, and more preferably 9 amino acids or more. The partial peptides may be useful for preparing antibodies against a protein of the invention, screening compounds binding to a protein of the present invention, or screening activators or inhibitors of a protein of the present invention. Alternatively, it may be used as an antagonist or competitive inhibitor for a protein of the invention. The partial peptides of the invention may be produced by genetic engineering techniques, well-known peptide synthesizing methods, or by excising a protein of the invention with a suitable peptidase. The solid-phase synthesizing method and the liquid-phase synthesizing method may be used as peptide synthesizing methods.

A DNA encoding a protein of this invention may be useful for producing the above proteins of the invention *in vivo* and *in vitro.* Furthermore, for example, it is also possible to use the DNAs for application to gene therapy and such of diseases arising from abnormalities of a gene encoding a protein of the present invention, and diseases that can be treated by the proteins of the invention. The DNAs of the invention may be provided in any form so long as it encodes a protein of the invention. Thus, the DNAs may be cDNAs synthesized from mRNAs, genomic DNAs, or chemically synthesized DNAs. Furthermore, a DNA comprising any nucleotide sequence based on the degeneracy of the genetic code may be included so long as it encodes a protein of the present invention.

The DNAs of the invention can be prepared by any method known to those skilled in the art. For example, a DNA may be prepared by constructing a cDNA library from cells expressing a protein of the present invention, and performing hybridization using a partial sequence of a DNA of the invention (e.g., SEQ ID NO: 1, 3 or 5) as a probe. A cDNA library may be constructed according to the method described in the literature (Sambrook J. et al., Molecular Cloning, Cold Spring Harbor Laboratory Press (1989)), or a commercial DNA library may be used. Alternatively, a DNA may be prepared by obtaining RNAs from a cell expressing a protein of the present invention, synthesizing cDNAs by a reverse transcriptase, synthesizing oligo DNAs based on the sequence of a present DNA (e.g., SEQ ID NO: 1, 3 or 5), performing PCR using the synthesized DNA as the primer, and amplifying the cDNA encoding a protein of the present invention.

By determining the nucleotide sequence of the obtained cDNA, the translation region encoded by the cDNA can be determined to obtain the amino acid sequence of a protein of the present invention. Furthermore, genomic DNA can be isolated by screening genomic DNA libraries using the obtained cDNA as a probe.

Specifically, this can be conducted as follows: First, mRNA is isolated from cells, tissues, and organs (for example, tissues, such as thyroid gland and testis; cancer cells, etc.) expressing a protein of the invention. Known methods can be used to isolate mRNA; for example, total RNA may be prepared by the guanidine ultracentrifugation method (Chirgwin, J.M. et al., Biochemistry 18: 5294-5299 (1979)), the AGPC method (Chomczynski, P. and Sacchi, N., Anal. Biochem. 162: 156-159 (1987)), and such, and mRNA may be purified from total mRNA using mRNA Purification Kit (Pharmacia) and such. Alternatively, mRNA may be directly prepared by QuickPrep mRNA Purification Kit (Pharmacia).

The obtained mRNA is used to synthesize cDNA using reverse transcriptase. cDNA may be synthesized using a kit, such as AMV Reverse Transcriptase First-strand cDNA Synthesis Kit (SEIKAGAKU CORPORATION). Alternatively, cDNA may be synthesized and amplified following the 5'-RACE method (Frohman, M.A. et al., Proc. Natl. Acad. Sci. U.S.A. 85: 8998-9002 (1988); Belyavsky, A. et al., Nucleic Acids Res. 17: 2919-2932 (1989)) that uses primers and such described herein; using 5'-Ampli FINDER RACE KIT (Clontech); and by polymerase chain reaction (PCR).

A desired DNA fragment is prepared from the obtained PCR products and ligated to a vector DNA. The recombination vector is used to transform *E. coli* and such, and the desired recombinant vector is prepared from a selected colony. The nucleotide sequence of the desired DNA can be verified by conventional methods, such as the dideoxy nucleotide chain termination method.

A DNA of the invention may be designed to have a sequence that is expressed more efficiently by taking into account the frequency of codon usage in the host used for expression (Grantham, R. et al., Nucleic Acids Research 9: r43-74 (1981)). The DNA of the invention may be also altered by a commercially available kit or a conventional method. For example, a DNA may be altered by digestion with restriction enzymes, insertion of a synthetic oligonucleotide or an appropriate DNA fragment, addition of a linker, or insertion of the start codon (ATG) and/or the stop codon (TAA, TGA, or TAG), and such.

Specifically, DNAs of the invention include a DNA consisting of the nucleotide sequence from the 154th "A" to 1110th "A" of SEQ ID NO: 1, 154th "A" to 2706th "T" of SEQ ID NO: 3, or 154th "A" to 2487th "T" of SEQ ID NO: 5.

The DNA of the present invention includes a DNA that hybridizes to a DNA comprising the nucleotide sequence of SEQ ID NO: 1, 3, or 5, wherein the DNA encodes a protein functionally equivalent to an above-mentioned protein of the present invention. Those skilled in the art can properly select appropriate conditions for hybridization, and specifically, the above-mentioned conditions can be used. Under these conditions, the higher the temperature, the higher the homology of the obtained DNA will be. The above hybridizing DNA is preferably a natural DNA, such as cDNA and chromosomal DNA.

Moreover, the present invention provides a vector containing a DNA of the invention as an insert. The vector may be useful for maintaining the DNA in host cells or producing a protein of the invention.

When *E.coli* (such as JM109, DH5α, HB101, and XL1Blue) is used as the host, there is no limitation other than that the vector should have an "ori" to amplify and mass-produce the vector in *E.coli;* and a marker gene for selecting the transformed *E.coli* (e.g., a drug resistance gene selected by a drug, such as ampicillin, tetracycline, kanamycin, or chloramphenicol). For example, M13-series vectors, pUC-series vectors, pBR322, pBluescript, pCR-Script, and so on can be used. Besides these vectors, pGEM-T, pDIRECT, pT7, and such can also be used for subcloning and excision of the cDNA as well. When a vector is used to produce a protein of the invention, an expression vector is especially useful. When the expression vector is expressed, for example, in *E*. *coli,* it should have the above characteristics in order to be amplified in *E. coli.* Additionally, when *E*. *coli,* such as JM109, DH5α, HB101, or XL1-Blue, are used as the host cell, the vector should have a promoter, e.g., the lacZ promoter (Ward et al., Nature 341: 544-546 (1989); FASEB J. 6: 2422-2427 (1992)), the araB promoter (Better et al., Science 240: 1041-1043) (1988), or the T7 promoter, that can efficiently promote the expression of the desired gene in *E. coli.* Other examples of the vectors are pGEX-5X-1 (Pharmacia), "QIAexpress system" (QIAGEN), pEGFP, pET (for this vector, BL21, a strain expressing T7 RNA polymerase, is preferably used as the host).

Further, the vector may contain a signal sequence that induces secretion of polypeptides. For producing proteins in the periplasm of *E. coli,* the pelB signal sequence (Lei S.P. et al., J. Bacteriol. 169: 4379 (1987)) may be used as the signal sequence for protein secretion. For example, the calcium chloride method or electroporation may be used to introduce the vector into host cells.

Example of vectors used to produce the proteins of the present invention include, for example, expression vectors other than those from *E. coli,* such as expression vectors derived from mammals (e.g., pcDNA3 (Invitrogen), pEGF-BOS (Nucleic Acids.Res., 18 (17): p5322 (1990)), pEF, and pCDM8); insect cells (e.g., "Bac-to-BAC baculovirus expression system" (GIBCO BRL), pBacPAK8); plants (e.g., pMH1 and pMH2); animal viruses (e.g., pHSV, pMV, and pAdexLcw); retroviruses (e.g., pZIPneo); yeasts (e.g., "Pichia Expression Kit" (Invitrogen), pNV1 and SP-Q01); and *Bacillus subtilis* (e.g., pPL608 and pKTH50).

In order to express the proteins in animal cells, such as CHO, COS, and NIH3T3 cells, the vector should include a promoter necessary for expression in such cells (e.g., the SV40 promoter (Mulligan et al., Nature 277: 108 (1979)), the MMLV-LTR promoter, the EF1α promoter (Mizushima et al., Nucleic Acids Res. 18: 5322 (1990)), and the CMV promoter). It is more preferable for the vector to additionally have a marker gene that enables selection of the transfected cells (for example, a drug resistance gene selected by a drug, such as neomycin and G418). Examples of vectors include pMAM, pDR2, pBK-RSV, pBK-CMV, pOPRSV, pOP13, and so on.

Furthermore, in order to stably express the gene and to amplify the copy number in cells, the method using CHO cells deficient in nucleic acid synthetic pathways as the host, incorporating into the CHO cells a vector (such as pCHOI) having a DHFR gene that compensates for the deficiency, and amplifying the vector with methotrexate (MTX) can be used. Furthermore, for transient gene expression, the method that transforms COS cells that have the gene for SV40 T-antigen on the chromosome with a vector (such as pCD) having the SV40 replication origin can be mentioned. The replication origins may be that of a polyomavirus, adenovirus, bovine papilomavirus (BPV), and the like. Further, to amplify the gene copy number in the host cells, selection markers, such as aminoglycoside transferase (APH) gene, thymidine kinase (TK) gene, *E. coli* xanthine guanine phosphoribosyl transferase (Ecogpt) gene, and dihydrofolate reductase (dhfr) gene may be comprised in the expression vector.

A DNA of the present invention can be expressed in animals by, for example, inserting the DNA of the invention into an appropriate vector and introducing the vector into a living body by the retrovirus method, the liposome method, the cationic liposome method, the adenovirus method, and so on. Thus, it is possible to perform gene therapy of diseases caused by a mutation in a Helicain gene. The vectors used in these methods include, but are not limited to, adenovirus vectors (such as pAdexlcw), retrovirus vectors (such as pZIPneo), and so on. General techniques for gene manipulation, such as insertion of a DNA of the present invention into a vector, can be performed according to conventional methods (Molecular Cloning, 5.61-5.63). Administration of the vector to the living body may be performed according to *ex vivo* methods or *in vivo* methods.

The present invention also relates to hosts into which a vector of this invention is introduced. The host cell into which the vector of the present invention is inserted is not particularly limited. For example, *E. coli,* various animal cells, and such can be used. The host cell of the present invention can be used, for example, as a production system to produce and express a protein of the invention. Protein production systems include *in vitro* and *in vivo* systems. Such production systems using eukaryotic cells or prokaryotic cells can be given as *in vitro* production systems.

As eukaryotic cells, for example, animal cells, plant cells, and fungal cells are available as hosts. Mammalian cells, such as CHO (J. Exp. Med., 108: 945 (1995)), COS, 3T3, myeloma, baby hamster kidney (BHK), HeLa, Vero; amphibian cells (e.g., *Xenopus* oocytes (Valle, et al., Nature 291: 338-340 (1981)); and insect cells (e.g., sf9, sf21, or Tn5) are known as animal cells. Among CHO cells, those deficient in the DHFR gene, dhfr-CHO (Proc. Natl. Acad. Sci. USA 77: 4216-4220 (1980)) and CHO K-1 (Proc. Natl. Acad. Sci. USA 60: 1275 (1968)), are particularly preferable. Among animal cells, CHO cells are particularly preferable for mass expression. A vector can be introduced into a host cell by, for example, the calcium phosphate method, the DEAE-dextran method, methods using cationic liposome DOTAP (Boehringer Mannheim), electroporation, lipofection, and so on.

As plant cells, for example, plant cells originating from *Nicotiana tabacum* are known as protein producing systems and may be used as callus cultures. As fungal cells, yeasts such as those of the *Saccharomyces* genus, for example, *Saccharomyces cerevisiae;* or filamentous bacteria, such as those belonging to *Aspergillus* genus, for example, *Aspergillus niger* are known.

Useful prokaryotic cells include bacterial cells. Bacterial cells, such as *E*. *coli,* for example, JM109, DH5α, HB101, and such, as well as *Bacillus subtilis* are known.

These cells are transformed by a desired DNA, and the resulting transformed cells are cultured *in vitro* to obtain the protein. For example, culture medium, such as DMEM, MEM, RPMI1640, or IMDM, may be used with or without serum supplements, such as fetal calf serum (FCS), as culture medium for animal cells. The pH of the culture medium is preferably between about 6 to 8. Such cells are typically cultured at about 30°C to 40°C, for about 15 to 200 hr, and the culture medium may be replaced, aerated, or stirred if necessary.

On the other hand, for example, production systems using animals and plants may be given as *in vivo* protein production systems. A desired DNA can be introduced into a plant or animal, and the protein is produced within the plant or animal, and then, the protein is recovered. The term "host" herein encompasses such animals and plants as well.

Animals to be used for the production systems described above include mammals and insects. Mammals, such as goats, pigs, sheep, mice, and bovine, may be used (Vicki Glaser, SPECTRUM Biotechnology Applications (1993)). Alternatively, the mammals may be transgenic animals.

For example, a desired DNA is prepared as a fusion gene with a gene such as goat β casein gene that encodes a protein intrinsically produced into milk. Next, the DNA fragment containing the fusion gene is injected into goat embryo, which are then implanted in female goat. The desired protein are then recovered from milk produced by the transgenic goats (i.e., those born from the goat that had received the modified embryo) or descendants thereof. To increase the amount of milk containing the protein produced by transgenic goats, appropriate hormone/hormones may be administered to the transgenic goats (Ebert, K.M. et al., Bio/Technology 12: 699-702 (1994)).

Alternatively, insects, such as silkworm, may be used. Baculoviruses into which a DNA encoding a desired protein has been inserted can be used to infect silkworms, and the desired protein can be recovered from the body fluid (Susumu, M. et al., Nature 315: 592-594 (1985)).

As plants, for example, tobacco can be used. When using tobacco, a DNA encoding a desired protein may be inserted into a plant expression vector, such as pMON 530, which is introduced into bacteria, such as *Agrobacterium tumefaciens.* Then, the bacteria can be used to infect tobacco, such as *Nicotiana tabacum*, and the desired polypeptide can be recovered from the leaves (Julian, K. -C. Ma et al., Eur. J. Immunol. 24: 131-138 (1994)).

A protein of the present invention obtained as above may be isolated from inside or outside of host (medium, etc.), and purified as a substantially pure homogenous protein. The method for protein isolation and purification is not limited to any specific method; in fact, any conventional isolation and purification methods may be used. For example, column chromatography, filtration, ultrafiltration, salting out, solvent precipitation, solvent extraction, distillation, immunoprecipitation, SDS-polyacrylamide gel electrophoresis, isoelectric focusing, dialysis, recrystalization, and such may be suitably selected and combined to isolate/purify the protein.

For example, affinity chromatography, ion exchange chromatography, hydrophobic chromatography, gel filtration, reversed-phase chromatography, adsorption chromatography, and such can be exemplified as chromatographies (Strategies for Protein Purification and Characterization: A Laboratory Course Manual. Ed Daniel R. Marshak et al., Cold Spring Harbor Laboratory Press (1996)). These chromatographies can be performed by liquid chromatography, such as HPLC, FPLC, and the like. The present invention encompasses proteins highly purified by such purification methods.

A protein may be optionally modified or partially deleted by treating it with appropriate protein-modifying enzymes before or after purification. For example, trypsin, chymotrypsin, lysylendopeptidase, protein kinase, glucosidase, and such are used as protein-modifying enzymes.

The present invention also provides antibodies binding to the protein of the invention. There is no particular restriction as to the form of the antibody of the present invention and the present invention includes polyclonal antibodies, as well as monoclonal antibodies. Antiserum obtained by immunizing animals such as rabbits with a protein of the present invention, as well as polyclonal and monoclonal antibodies of all classes, human antibodies, and humanized antibodies made by genetic engineering, are included.

A protein of the invention that is used as a sensitizing antigen for obtaining antibodies is not restricted by the animal species from which it is derived, but is preferably a protein derived from mammals, for example, humans, mice, or rats, especially preferably from humans. Proteins of human origin can be obtained by using the nucleotide sequence or amino acid sequence disclosed herein.

Herein, an intact protein or its partial peptide may be used as the antigen for immunization. As partial peptides of the proteins, for example, the amino (N) terminal fragment of the protein, and the carboxy (C) terminal fragment can be given. "Antibody" as used herein refers to antibodies that specifically react with the full-length or fragments of the protein.

A gene encoding a protein of the invention or a fragment thereof is inserted into a well-known expression vector, and by transforming the host cells with the vector described herein, the objective protein or a fragment thereof is obtained from inside or outside the host cell using well-known methods, and this protein can be used as the sensitizing antigen. Alternatively, cells expressing the protein, cell lysates, or chemically synthesized proteins of the invention may be also used as a sensitizing antigen. Short peptides are preferably used as antigens by appropriately combining them with carrier proteins, such as keyhole limpet hemocyanin, bovine serum albumin, and ovalbumin.

The mammals that are immunized by the sensitizing antigen are not restricted, but it is preferable to select animals by considering the adaptability with the parent cells used in cell fusion. Generally, animals belonging to the rodentia, lagomorpha, and Primate family are used.

Examples of animals belonging to rodentia that may be used include mice, rats, hamsters, and such. Examples of animals belonging to lagomorpha that may be used include, for example, rabbits. Examples of Primates that may be used include monkeys. Examples of monkeys to be used include the infraorder catarrhini (Old World Monkeys), for example, cynomolgus monkeys, rhesus monkeys, sacred baboons, chimpanzees, and such.

Well-known methods may be used to immunize animals with the sensitizing antigen. For example, the sensitizing antigen is generally injected intraperitoneally or subcutaneously into mammals. Specifically, the sensitizing antigen is suitably diluted and suspended in physiological saline or phosphate-buffered saline (PBS) and mixed with a suitable amount of general adjuvant if desired, for example, with Freund's complete adjuvant. Then, the solution is emulsified and injected into the mammal. Thereafter, the sensitizing antigen suitably mixed with Freund's incomplete adjuvant is preferably given several times every 4 to 21 days. A suitable carrier can also be used when immunizing an animal with the sensitizing antigen. After the immunization, the elevation in the level of serum antibody is detected by usual methods.

Polyclonal antibodies against a protein of the invention can be obtained as follows. After verifying that a desired serum antibody level has been reached, blood is withdrawn from the mammal sensitized with the antigen. Serum is isolated from this blood using well-known methods. The serum containing the polyclonal antibody may be used as the polyclonal antibody, or according to needs, the polyclonal antibody-containing fraction may be further isolated from the serum. For instance, a fraction of antibodies that specifically recognize the protein of the invention may be prepared using an affinity column to which the protein is coupled. Then, the fraction may be further purified by a Protein A or Protein G column in order to prepare immunoglobulin G or immunoglobulin M.

To obtain monoclonal antibodies, after verifying that the desired serum antibody level has been reached in the mammal sensitized with the above-described antigen, immunocytes are taken from the mammal and used for cell fusion. For this purpose, splenocytes can be mentioned as preferable immunocytes. As parent cells fused with the above immunocytes, mammalian myeloma cells are preferably used. More preferably, myeloma cells that have acquired the feature, which can be used to distinguish fusion cells by agents, are used as the parent cell.

The cell fusion between the above immunocytes and myeloma cells can be conducted according to known methods, for example, the method of Milstein et al. (Galfre, G. and Milstein, C., Methods Enzymol. 73: 3-46 (1981)).

The hybridoma obtained from cell fusion is selected by culturing the cells in a standard selective culture medium, for example, HAT culture medium (hypoxanthine, aminopterin, thymidine-containing culture medium). The culture in this HAT medium is continued for a period sufficient enough for cells (non-fusion cells) other than the objective hybridoma to perish, usually from a few days to a few weeks. Next, the usual limiting dilution method is carried out, and the hybridoma producing the objective antibody is screened and cloned.

Other than the above method for obtaining hybridomas, by immunizing an animal other than humans with the antigen, a hybridoma producing the objective human antibodies having the activity to bind to proteins can be obtained by the method of sensitizing human lymphocytes, for example, human lymphocytes infected with the EB virus, with proteins, protein-expressing cells, or lysates thereof in vitro, fusing the sensitized lymphocytes with myeloma cells derived from human, for example U266, having a permanent cell division ability (Unexamined Published Japanese Patent Application (JP-A) No. Sho 63-17688).

Subsequently, the hybridomas thus obtained are transplanted into the abdominal cavity of a mouse from which the ascites is collected. The obtained monoclonal antibodies can be purified by, for example, ammonium sulfate precipitation; protein A or protein G column; DEAE ion exchange chromatography; an affinity column to which the protein of the present invention is coupled; and so on. The antibody may be useful for the purification or detection of a protein of the invention. It may also be a candidate for an agonist or antagonist of the protein. Furthermore, it is possible to use it for antibody treatment of diseases in which the protein is implicated. For *in vivo* administration (in such antibody treatment), human antibodies or humanized antibodies may be favorably used because of their reduced immunogenicity.

For example, a human antibody against a protein can be obtained using hybridomas made by fusing myeloma cells with antibody-producing cells obtained by immunizing a transgenic animal comprising a repertoire of human antibody genes with an antigen, such as proteins, protein-expressing cells, or cell lysates thereof (WO92/03918, WO93/2227, WO94/02602, WO94/25585, WO96/33735, and WO96/34096).

Other than producing antibodies by using hybridoma, antibody-producing immunocytes, such as sensitized lymphocytes that are immortalized by oncogenes, may also be used.

Such monoclonal antibodies can also be obtained as recombinant antibodies produced by the genetic engineering technique (for example, Borrebaeck, C.A.K. and Larrick, J.W., THERAPEUTIC MONOCLONAL ANTIBODIES, Published in the United Kingdom by MACMILLAN PUBLISHERS LTD, (1990)). Recombinant antibodies are produced by cloning the encoding DNA from immunocytes, such as hybridoma or antibody-producing sensitized lymphocytes, incorporating this into a suitable vector, and introducing this vector into a host to produce the antibody. The present invention encompasses such recombinant antibodies as well.

Moreover, the antibody of the present invention may be an antibody fragment or a modified-antibody so long as it binds to a protein of the invention. For example, Fab, F (ab')₂, Fv, or single chain Fv in which the H chain Fv and the L chain Fv are suitably linked by a linker (scFv, Huston, J.S. et al., Proc. Natl. Acad. Sci. U.S.A. 85: 5879-5883 (1988)) can be given as antibody fragments. Specifically, antibody fragments are produced by treating antibodies with enzymes, for example, papain, pepsin, and such, or by constructing a gene encoding an antibody fragment, introducing this into an expression vector, and expressing this vector in suitable host cells (for example, Co, M.S. et al., J. Immunol. 152: 2968-2976 (1994); Better, M. and Horwitz, A.H., Methods Enzymol. 178: 476-496 (1989); Pluckthun, A. and Skerra, A., Methods Enzymol. 178: 497-515 (1989); Lamoyi, E., Methods Enzymol. 121: 652-663 (1986); Rousseaux, J. et al., Methods Enzymol. 121: 663-669 (1986); Bird, R.E. and Walker, B.W., Trends Biotechnol. 9: 132-137 (1991)).

As modified antibodies, antibodies bound to various molecules, such as polyethylene glycol (PEG), can be used. The antibodies of the present invention encompass such modified antibodies as well. To obtain such a modified antibody, chemical modifications are done to the obtained antibody. These methods are already established in the field.

The antibody of the invention may be obtained as a chimeric antibody, comprising non-human antibody-derived variable region and human antibody-derived constant region, or as a humanized antibody comprising non-human antibody-derived complementarity determining region (CDR), human antibody-derived framework region (FR), and human antibody-derived; constant region by conventional methods.

Antibodies thus obtained can be purified to uniformity. The separation and purification methods used in the present invention for separating and purifying an antibody may be any method usually used for proteins. For instance, column chromatography, such as affinity chromatography; filter; ultrafiltration; salt precipitation; dialysis; SDS-polyacrylamide gel electrophoresis; isoelectric point electrophoresis; and so on, may be appropriately selected and combined to isolate and purify the antibodies (Antibodies: a laboratory manual. Ed Harlow and David Lane, Cold Spring Harbor Laboratory (1988)), but is not limited thereto. Antibody concentration of the above mentioned antibodies can be assayed by measuring the absorbance, or by the enzyme-linked immunosorbent assay (ELISA), etc.

Protein A or Protein G column can be used for the affinity chromatography. Protein A column may be, for example, Hyper D, POROS, Sepharose F.F. (Pharmacia), and so on.

Other chromatography may also be used, such as ion exchange chromatography, hydrophobic chromatography, gel filtration, reverse phase chromatography, and adsorption chromatography (Strategies for Protein Purification and Characterization: A laboratory Course Manual. Ed. by Marshak D.R. et al., Cold Spring Harbor Laboratory Press (1996)). These may be performed on liquid chromatography, such as HPLC or FPLC.

Examples of methods for measuring the antigen-binding activity of the antibodies of the invention include, for example, measurement of absorbance, enzyme-linked immunosorbent assay (ELISA), enzyme immunoassay (EIA), radio immunoassay (RIA), or fluorescent antibody method. For example, when using ELISA, a protein of the invention is added to a plate coated with the antibodies of the invention, and next, the objective antibody sample, for example, culture supernatants of antibody-producing cells, or purified antibodies are added. Then, secondary antibody recognizing the antibody, which is labeled by alkaline phosphatase and such enzymes, is added; the plate is incubated and washed; and the absorbance is measured to evaluate the antigen-binding activity after adding an enzyme substrate, such as *p*-nitrophenyl phosphate. As the protein, a protein fragment, for example, a fragment comprising a C-terminus, or a fragment comprising an N-terminus may be used. To evaluate the activity of an antibody of the invention, BIAcore (Pharmacia) may be used.

According to these methods, an antibody of the invention and a sample presumed to contain a protein of the invention are contacted, and the protein of the invention is detected or assayed by detecting or assaying the immune complex of the above-mentioned antibody and protein. A method of detecting or assaying a protein of the invention is useful in various experiments using proteins as it can specifically detect or assay the proteins. It is also useful in the examination of cancer as described below.

Another object of this invention is to provide a polynucleotide having at least 15 nucleotides that is complementary to a DNA encoding a human Helicain protein (SEQ ID NO: 1, 3 or 5) or its complementary strand.

Herein, the term "complementary strand" is defined as one strand of a double stranded DNA composed of A:T (in the case of RNA, U) and G:C base pairs to the other strand. Also, "complementary" is defined as not only those completely matching within a continuous region of at least 15 nucleotides, but also having a homology of at least 70%, favorably 80% or higher, more favorably 90% or higher, and most favorably 95% or higher within that region. The homology may be determined using the algorithm described herein.

Probes or primers for detection or amplification of a DNA encoding a protein of the invention, or a nucleotide or nucleotide derivative for the suppression of the protein expression (such as antisense oligonucleotide and ribozyme, or DNAs encoding them, etc.) are included in such nucleic acids. Such nucleic acids may be also used for preparing DNA chips.

When used as primers, such nucleic acids are complementary at the 3' end, and restriction enzyme recognition sequences or tags can be added to the 5' end.

The antisense oligonucleotide that hybridizes with a portion of the nucleotide sequence of any of SEQ ID NO: 1, 3, and 5 is also included in the antisense oligonucleotides of the present invention. This antisense oligonucleotide is preferably one against at least 15 continuous nucleotides in any one of the nucleotide sequences of SEQ ID NO: 1, 3 and 5. More preferably, it is the antisense oligonucleotide against at least 15 continuous nucleotides containing a translation start codon.

Derivatives or modified products of antisense oligonucleotides can be used as antisense oligonucleotides. Examples of such modified products include, for example, lower alkyl phosphonate modifications, such as methyl-phosphonate-type or ethyl-phosphonate-type; phosphorothioate; and phosphoroamidate-modified products.

The term "antisense oligonucleotide(s)" as used herein refers to, not only those in which the nucleotides corresponding to those constituting a specified region of a DNA or mRNA are entirely complementary, but also those having a mismatch of one or more nucleotides, so long as the DNA or mRNA and the oligonucleotide can selectively and stably hybridize with the nucleotide sequence of SEQ ID NO: 1, 3 or 5.

An antisense oligonucleotide derivative of the present invention acts upon cells producing a protein of the invention by binding to the DNA or mRNA encoding the protein to inhibit its transcription or translation, and to promote the degradation of mRNA, and has an effect of suppressing the function of the protein of the invention by suppressing the expression of the protein.

An antisense oligonucleotide derivative of the present invention can be made into an external preparation, such as a liniment and a poultice, by mixing with a suitable base material, which is inactive against the derivatives.

Also, as needed, the derivatives can be formulated into tablets, powders, granules, capsules, liposome capsules, injections, solutions, nose-drops, and freeze-dried agents by adding excipients, isotonic agents, solubilizers, stabilizers, preservatives, pain-killers, etc. These can be prepared using conventional methods.

An antisense oligonucleotide derivative of the present invention is given to the patient by directly applying onto the ailing site, by injecting into the blood vessel and such, so that it will reach the ailing site. An antisense-mounting material can be also used to increase durability and membrane-permeability. Examples are, liposome, poly-L lysine, lipid, cholesterol, lipofectin, or derivatives of them.

The dosage of an antisense oligonucleotide derivative of the present invention can be adjusted suitably according to the patient's condition and used in desired amounts. For example, a dose range of 0.1 to 100 mg/kg, preferably 0.1 to 50 mg/kg can be administered.

The antisense oligonucleotide derivatives of the present invention are useful in inhibiting the expression of the proteins of the invention, and therefore are useful in suppressing the biological activity of the proteins of the invention. Also, expression-inhibitors comprising the antisense oligonucleotide derivatives of the present invention are useful, because of their capability to suppress the biological activity of the proteins of the invention.

Antibodies of the present invention and polynucleotides of the present invention are useful for examining for cancers. In cancer cells, expression levels of the "Helicain" genes of the present invention were significantly elevated, which indicates that they may be used as markers for cancer. For example, the existence of cancer cells and/or the progress of cancer can be examined by detecting, in the test sample, the proteins of the present invention or mRNAs encoding the proteins of the invention. When a significantly higher expression of a "Helicain" gene compared with non-cancer cells is detected in a cell derived from subjects of the examination, the cell is suspected to be a cancer cell. The detection of mRNA can be conducted by Northern hybridization, RT-PCR, and such. Alternatively, the detection may be performed by DNA chip. The detection of a protein can be conducted by immunoprecipitation, Western blotting, immunohistochemistry, ELISA, etc. using an antibody of the present invention.

In the case of using an antibody of the present invention as a reagent for examination, it may be combined properly with solutes, such as stabilizers, salts, buffers, and such; or solvents, such as water, saline, and such.

Proteins of this invention are useful for screening compounds binding to the- proteins. Specifically, the proteins are used in the method of screening for compounds that bind to the proteins of this invention, in which the method comprises bringing a protein of this invention into contact with a test sample that is expected to contain a compound to bind to the protein; and selecting the compound binding to the protein of this invention.

Proteins of this invention used in the screening may be any of recombinant, natural or partial peptides. Also they may be purified proteins, partial peptides thereof, or in the form of proteins expressed on the cell surface or membrane fractions. Samples to be tested are not limited, but may be cell extracts, culture supernatants, fermented products of microorganisms, extracts of marine organisms, plant extracts, purified or partially purified proteins, peptides, non-peptide compounds, synthetic low molecular compounds, or natural compounds. A protein of the invention may be exposed to the sample as a purified protein or soluble protein, in a form bound to a support, as a fusion protein with other protein(s), in a form expressed on the surface of cell membrane, or as membrane fractions.

A protein of the invention may be used to screen for proteins that bind to the protein using a variety of methods known to those skilled in the art. These screening can be carried out, for example, by the immunoprecipitation method. Specifically, the method can be carried out as follows. A gene encoding a protein of this invention is expressed by inserting the gene into a vector for foreign gene expression, such as pSV2neo, pcDNA I, and pCD8, and expressing the gene in animal cells, etc. Any generally used promoters may be employed for the expression, including the SV40 early promoter (Rigby In Williamson (ed.), Genetic Engineering, Vol. 3. Academic Press, London, p.83-141 (1982)), the EF-1 α promoter (Kim, et al. Gene 91: 217-223 (1990)), the CAG promoter (Niwa, et al. Gene 108: 193-200 (1991)), the RSV LTR promoter (Cullen, Methods in Enzymology 152: 684-704 (1987)), the SR α promoter (Takebe et al., Mol. Cell. Biol. 8: 466 (1988)), the CMV immediate early promoter (Seed and Aruffo Proc. Natl. Acad. Sci. USA 84: 3365-3369 (1987)), the SV40 late promoter (Gheysen and Fiers J. Mol. Appl. Genet. 1: 385-394 (1982)), the Adenovirus late promoter (Kaufman et al., Mol. Cell. Biol. 9: 946 (1989)), the HSV TK promoter, etc. Transfer of a foreign gene into animal cells for its expression can be performed by any of the following methods, including the electroporation method (Chu, G. et al., Nucl. Acid Res. 15: 1311-1326 (1987)), the calcium phosphate method (Chen, C. and Okayama, H. Mol. Cell. Biol. 7: 2745-2752 (1987)), the DEAE dextran method (Lopata, M. A. et al. Nucl. Acids Res. 12: 5707-5717 (1984); Sussman, D. J. and Milman, G. Mol. Cell. Biol. 4: 1642-1643 (1985)), the lipofectin method (Derijard, B. Cell. 7: 1025-1037 (1994); Lamb, B. T. et al. Nature Genetics 5: 22-30 (1993)), Rabindran, S. K. et al. Science 259: 230-234 (1993)), etc. A protein of the present invention can be expressed as a fusion protein having the recognition site for a monoclonal antibody by introducing a recognition site (epitope) for a monoclonal antibody, the specificity of which has been established, into the N- or C-terminal of the protein of this invention. For this purpose, a commercial epitope-antibody system can be utilized (Jikken Igaku (Experimental Medicine) 13: 85-90 (1995)). Vectors are commercially available which express fusion proteins with the β-galactosidase, the maltose-binding protein, the glutathione S-transferase, the green fluorescence protein (GFP), and such, via the multi-cloning site.

To minimize the alteration of the properties of a protein of this invention due to the formation into a fusion protein, a method has been reported to prepare a fusion protein by introducing only a small epitope portion comprising several to a dozen amino acid residues. For example, the epitopes of polyhistidine (His-tag), influenza hemagglutinin (HA), human c-myc, FLAG, Vesicular stomatitis virus glycoprotein (VSV-GP), T7 gene 10 protein (T7-tag), human herpes simplex virus glycoprotein (HSV-tag), E-tag (epitope on the monoclonal phage), and such, and monoclonal antibodies to recognize these epitopes can be utilized as the epitope-antibody system for screening proteins binding to the protein of this invention (Jikken Igaku (Experimental Medicine) 13, 85-90 (1995)).

During immunoprecipitation, immune complexes are formed by adding these antibodies to the cell lysate prepared using suitable surfactants. This immune complex consists of a protein of this invention, a protein capable of binding to the protein, and an antibody. The immunoprecipitation can also be performed using an antibody to a protein of this invention besides antibodies to the above-described epitopes. An antibody to a protein of this invention can be prepared by inserting a gene encoding a protein of this invention into an appropriate expression vector for *E*. *coli* to express it in the bacterium, purifying the protein thus expressed, and immunizing rabbits, mice, rats, goats, chicken; and such, with the purified protein. The antibody can also be prepared by immunizing the above-described animals with partial peptides of a protein of this invention.

Immune complexes can be precipitated using, for example, Protein A Sepharose and Protein G Sepharose in case where the antibody is a murine IgG antibody. In addition, in the case where a protein of this invention is prepared as a fusion protein with the epitope of, for example, GST, and such, the immune complex can be formed using a substance that specifically binds to these epitopes, such as glutathione-Sepharose 4B, and such, giving the same result as in the case where the antibody for the protein of this invention is used.

Immune precipitation, in general, may be carried out according to, or following the method described in the literature (Harlow, E. and Lane, D.: Antibodies, pp.511-552, Cold Spring Harbor Laboratory publications, New York (1988)).

SDS-PAGE is generally used for the analysis of immunoprecipitated proteins, and bound proteins can be analyzed based on the molecular weights of proteins using a gel of an appropriate concentration. In this case, although proteins bound to a protein of this invention, in general, are hardly detectable by the usual protein staining method, such as Coomassie staining and silver staining, the detection sensitivity can be improved by culturing cells in a medium containing the radio isotope-labeled ³⁵S-methionine and ³⁵S-cysteine to label proteins inside the cells, and detecting the labeled proteins. Once the molecular weight of the protein is determined, the desired protein can be purified directly from SDS-polyacrylamide gel and sequenced.

In addition, screening of proteins binding to a protein of the present invention can be also performed using the West-western blotting method (Skolnik, E. Y. et al., Cell 65: 83-90 (1991)). Specifically, cDNA is isolated from cells, tissues and organs (for example, thyroid gland, testis, cancer cells, etc.) in which a protein binding to the protein of this invention is expected to be expressed, and transferred into a phage vector (for example, λgt11, ZAPII etc.) to prepare a cDNA library, which is then expressed on plates coated with a growth medium. The protein thus expressed is fixed on a filter, which is then reacted with the labeled, purified protein of this invention, and plaques expressing proteins bound to the protein of this invention can be detected by the label. Methods for labeling a protein of this invention include methods utilizing the binding activity of biotin and avidin, methods utilizing antibodies specifically binding to the protein of this invention, or peptides or polypeptides (for example, GST etc.) fused with the protein of this invention, methods utilizing the radioisotopes, methods utilizing fluorescence, etc.

Further, another embodiment of the screening method of this invention is exemplified by a method utilizing the 2-hybrid system using cells (Fields, S., and Sternglanz, R., Trends. Genet. 10: 286-292 (1994); Dalton S, and Treisman R, "Characterization of SAP-1, a protein recruited by serum response factor to the c-fos serum response element." Cell 68: 597-612 (1992); "MATCHMARKER Two-Hybrid System", "Mammalian MATCHMARKER Two-Hybrid Assay Kit",_{,} and "MATCHMARKER One-Hybrid System" (Clonetech); "HybriZAP Two-Hybrid Vector system" (Stratagene)). In the two-hybrid system, a protein of the invention or partial peptide thereof may be fused to the DNA binding domain of SRF or GAL4, and expressed in yeast. A cDNA library is constructed from cells predicted to express proteins that bind to the protein of the present invention, wherein the cDNA library is constructed in such a way that the proteins are expressed as fusion proteins with transcription activation regions of VP16 or GAL4. The cDNA library is transfected into the above yeast, and then positive clones are detected to isolate the cDNA derived from the library (Expression of a protein that binds to the protein of the invention in yeast leads to the binding of the two proteins, and results in the activation of the reporter gene, which allows to detect positive clones). The protein encoded by the isolated cDNA may be obtained by introducing the cDNA into *E.coli* and expressing it therein. Thus, it is possible to prepare proteins that binds to a protein of the invention and genes encoding them. The reporter gene used in the two-hybrid system include HIS3, Ade2, LacZ, CAT, luciferase, PAI-I (plasminogen activator inhibitor type I), and so on, but is not limited thereto.

Screening for compounds, which bind to a protein of this invention, can be also carried out using affinity chromatography. For example, the protein of this invention is immobilized on a carrier in the affinity chromatography column, to which a test sample, which is expected to express a protein binding to the protein of this invention, is applied. Samples may be cell extracts, cell lysates, or else. After applying the test sample, the column is washed, and proteins that bind to the protein of the invention can be obtained.

The obtained protein may be analyzed for its amino acid sequence to synthesize oligonucleotide probes, which may be used to screen a cDNA library to obtain a DNA encoding the protein.

A biosensor that utilizes surface plasmon resonance may be used to detect or measure the bound compound. Such sensor (as BIAcore (Pharmacia)) may enable to observe the interaction at real time using a small amount of protein without the need of labeling. Thus, it is possible to assess the interaction between a protein of the invention and samples using biosensor such as BIAcore.

Moreover, compounds that bind to a protein of the invention (including agonists and antagonists), which compounds are not always proteins, may be isolated using a variety of methods known to those skilled in the art. For example, the protein of the invention may be fixed and exposed to synthetic compounds, a bank of natural compounds, or a random phage peptide library to screen a molecule that binds to the protein. Alternatively, high throughput screening using combinatorial chemistry may be performed (Wrighton NC, Farrel FX, Chang R, Kashyap AK, Barbone FP, Mulcahy LS, Johnson DL, Barrett RW, Jolliffe LK, Dower WJ., "Small peptides as potent mimetics of the protein hormone erythropoietin." Science (UNITED STATES) 273: 458-464 (Jul 26 1996); Verdine G.L., "The combinatorial chemistry of nature." Nature (ENGLAND) 384: 11-13 (Nov 7 1996); Hogan JC Jr., "Directed combinatorial chemistry." Nature (ENGLAND) 384: 17-9 (Nov 7 1996)).

Compounds that bind to a protein of the present invention serve as a candidate for agents that regulate the activity of the protein of the present invention; and may be applicable to the therapy of diseases originating from abnormalities in the expression, function, and such of the protein of the present invention, or diseases, which can be treated by regul'ating the activity of the protein of the present invention. Substances, wherein a portion of the structure of a compound, which can be obtained by the method for screening of the present invention having the activity to bind to a protein of the present invention, is modified by addition, deletion and/or substitution, are included as compounds binding to the protein of the present invention.

Moreover, the present invention provides a method of screening for a compound that regulates the binding between a "Helicain" protein of the present invention and Tesmin protein (Sugihara, T. et al., 1999, Genomics 57: 130-136). The method of screening for a compound includes the steps of: (a) contacting the protein of the present invention and Tesmin in the presence of a test sample; (b) detecting the binding between the protein of the present invention and Tesmin; and (c) selecting the compound that inhibits or promotes the binding compared with that observed in the absence of test samples.

The form of proteins used in the screening is not limited so long as the binding activity between the two proteins or the complex forming ability are retained. Thus, they don't have to be full-length proteins, and may be mutant or partial peptides. Moreover, they may be natural proteins or recombinants. Furthermore, they may be fusion proteins with other peptide(s). These proteins can be produced similarly to the method of screening for compounds binding to a protein of the present invention described above. Further, the test samples are not limited, and include, for example, culture supernatants of cells, products of ferment microorganisms, extracts of oceanic lives, plant extracts, prokaryotic cell extracts, eucaryotic monad extracts or animal cell extracts; or libraries thereof; purified or crude proteins; peptides; non-peptidic compounds; synthetic low molecular weight compounds; and natural compounds. The proteins of the present invention may be contacted with the test sample in the form, for example, as purified proteins, soluble proteins, carrier-bound forms, fusion proteins with other protein(s), expressed on cell membrane, or membrane fractions.

Such screening can be conducted, for example, by two-hybrid method using yeast or animal cells. Specifically, inhibitors or promoters can be screened, using luciferase activity as an index, by integrating a Helicain gene and Tesmin gene into the pVP16 vector and the pM vector, respectively, and co-expressing the GAL4 luciferase vector in the cell.

Further, the screening can be conducted, for example, by immunoprecipitation. The binding of a Helicain and Tesmin can be determined by incubating the Helicain and Tesmin under the existence of a test sample; recovering the resultant complex using antibody against one of the proteins or the tag fused to one of the proteins; and detecting the other protein using antibody against the protein and such. The detection can be facilitated by labeling the other protein with a different tag and such. Alternatively, one of the proteins may be bound to a support and the other protein is bound thereto to apply the test sample. The effect of the test sample can be assayed by detecting whether the bound protein dissociates or not. Moreover, screening can be conducted by ELISA.

Furthermore, screening using surface plasmon resonance as described above for the screening of proteins binding to Helicain, high-throughput screening using combinatorial chemistry, and so on, may be also used.

For example, compounds regulating the binding of proteins with Helicain proteins, other than the binding of Tesmin and Helicain proteins, can be also screened similarly to the screening method described above.

Compounds that regulate the binding of a protein of the present invention and Tesmin serve, for example, as a candidate for agents that inhibit or promote the activity of the protein of the present invention; and may be applicable for the therapy of diseases originating from abnormalities in the expression, function, and such of the protein of the present invention, or diseases that can be treated by regulating the activity of the protein of the present invention. Substances, wherein the partial structure of compounds obtained by the screening method of the present invention is modified by the addition, deletion and/or substitution, are also included in the present invention.

Further, the present invention also provides a method of screening for compound that regulate RNA helicase activity of a "Helicain" protein of the present invention. The method of screening for a compound includes the steps of: (a) contacting a double stranded nucleic acid as a substrate to the protein in the presence of a test sample; (b) detecting the dissociation of single stranded RNA from the double stranded nucleic acid; and (c) selecting the compound that suppresses or promotes the dissociation compared with that observed in the absence of the test sample.

The form of the protein used in the screening is not limited so long as it retains RNA helicase activity, and may be mutant or partial peptides besides being a full-length protein. Moreover, the protein may be a natural protein or recombinant. Furthermore, the protein may be a fusion protein with other peptide(s). Such proteins can be produced similarly to the case for screening of a compound binding to a protein of the present invention described above. Further, no limitation exist for the test sample and includes, for example, culture supernatants of cells, products of ferment microorganisms, extracts of oceanic lives, plant extracts, prokaryotic cell extracts, eucaryotic monad extracts or animal cell extracts; libraries thereof; purified or crude proteins; peptides; non-peptidic compounds; synthetic low molecular weight compounds; and natural compounds. The protein of the present invention to which a test sample is contacted can be in the form, for example, of purified protein, soluble protein, carrier-bound protein, fusion protein with other protein(s), protein expressed on plasma membrane, and membrane fraction.

According to the screening described above, for example, a single stranded RNA is fixed on a plate, and a complementary RNA labeled with Digoxigenin is bound to the fixed RNA to prepare a double stranded RNA. The sequence of the RNA strand is not limited. However, a length of around 40 bp is preferable. A Helicain protein is added thereto in the existence or absence of a test sample, the amount of labeled RNA strand on the plate is measured by ELISA method and such, and the activity is measured as the decrease of the amount of labeled RNA (Hsu, C.C. et al., 1998, Biochem. Biophys. Res. Commun. 253: 594-599). Then, the compound that suppresses or promotes the activity compared with that observed in the absence of the test sample (control) is selected. Alternatively, the screening may be performed by a method measuring the activity on PAGE gel by labeling the nucleic acid with radioisotopes gel (Tai, C.L. et al., 1996, J. Virol. 70: 8477-8484).

Further, the present invention provides a method of screening for a compound that regulates the localization to the nucleolus of a protein of the present invention. The screening utilizes the fact that the proteins of the present invention localize into the nucleolus as an index, and comprises the steps of: (a) contacting a test sample with or introducing a test sample into cells expressing the protein; (b) detecting the cellular localization of the protein; and (c) selecting the compound that suppresses or promotes the localization into the nucleolus of the protein compared with that observed in the absence of the test sample. The proteins used for the screening don't have to be full-length proteins so long as they have the activity to localize into the nucleolus. Furthermore, tags or other proteins can be fused thereto, *ad libitum.* For example, as described in the Examples, the intranuclear localization can be easily detected when expressed as a fusion protein with GFP. The test sample is not limited in any way, as described above.

Compounds that regulate RNA helicase activity of a protein of the present invention and those regulating the localization of a protein of the present invention may be applied for the therapy for diseases originating from abnormalities of expression, function, and such of the protein of the present invention, or diseases that may be treated by regulating the activity of the protein of the present invention. Specifically, compounds that suppress the activity or expression of the protein of the present invention are expected to be applicable as anticancer agents, since elevated expression of the proteins of the present invention is observed in cancer cells. Substances, wherein the partial structure of the compounds obtained by the method for screening of the present invention described above is modified by the addition, deletion and/or substitution, are also included in the present invention.

In the case of using compounds that binds to a protein of the present invention or that regulate the activity of a protein of the present invention, which compounds can be isolated by the screening of the present invention, as a medicine for human and mammals, for example, mouse, rat, guinea pig, rabbit, chicken, cat, dog, sheep, pig, bovine, monkey, baboon, or chimpanzee, it can be administrated as pharmaceutical compositions prepared by well known pharmaceutical preparation methods besides direct administration of the protein or the isolated compound itself to the patient. For example, according to the need, the drugs can be taken orally, as sugar-coated tablets, capsules, elixirs, and microcapsules; or parenterally, in the form of injections of sterile solutions, suspensions with water, or any other pharmaceutically acceptable liquid. For example, the compounds can be mixed with pharmacologically acceptable carriers or medium, specifically, sterilized water; physiological saline; plant-oil; emulsifiers; solvents; surfactants; stabilizers; flavoring agents; excipients; vehicles; preservatives; and binders, in a unit dose form required for generally accepted drug implementations. The amount of active ingredients in these preparations makes a suitable dosage acquirable within the indicated range.

Examples for additives which can be mixed to tablets and capsules are: binders, such as gelatin, corn starch, tragacanth gum, and gum arabic; excipients, such as crystalline cellulose; swelling agents, such as corn starch, gelatin, and alginic acid; lubricants, such as magnesium stearate; sweeteners, such as sucrose, lactose, and saccharin; flavoring agents, such as peppermint, Gaultheria adenothrix oil, and cherry. 'When the unit dosage form is a capsule, a liquid carrier, such as oil, can also be included in the above ingredients. Sterile composites for injections can be formulated following normal drug implementations using vehicles, such as distilled water used for injections.

For example, physiological saline, glucose, and other isotonic liquids including adjuvants, such as D-sorbitol, D-mannnose, D-mannitol, and sodium chloride, can be used as aqueous solutions for injections. These can be used in conjunction with suitable solubilizers, such as alcohol, specifically ethanol, polyalcohols such as propylene glycol and polyethylene glycol; non-ionic surfactants, such as Polysorbate 80 (TM) and HCO-50.

Sesame oil or Soy-bean oil can be used as a oleaginous liquid and may be used in conjunction with benzyl benzoate or benzyl alcohol as solubilizers; may be formulated with buffer, such as phosphate buffer and sodium acetate buffer; pain-killers, such as procaine hydrochloride; stabilizers, such as benzyl alcohol, phenol; and anti-oxidants. The prepared injection is generally filled into a suitable ampule.

Methods well known to those skilled in the art may be used to administer the pharmaceutical compound to patients, for example, as intraarterial, intravenous, percutaneous injections and also as intranasal, transbronchial, intramuscular or oral administrations. The dosage and method for administration vary according to the body-weight and age of the patient, the administration method, and such; but one skilled in the art can suitably select them. If said compound is encodable by a DNA, said DNA can be inserted into a vector for gene therapy to perform the therapy. The dosage and method for administration vary according to the body-weight, age, symptoms of a patient, and so on, but one skilled in the art can suitably select them.

Although there are some differences according to the symptoms, the dose of a compound that binds with a protein of the present invention, or a compound that regulates the activity of a protein of this invention is about 0.1 mg to about 100 mg per day, preferably about 1.0 mg to about 50 mg per day, and more preferably about 1.0 mg to about 20 mg per day, when administered orally to a standard adult (weight 60 kg).

When the protein is administered parenterally in the form of an injection to a standard adult (weight 60 kg), although there are some differences according to the patient, target organ, symptoms, and method of administration, it is convenient to intravenously inject a dose of about 0.01 mg to about 30 mg per day, preferably about 0.1 to about 20 mg per day, and more preferably about 0.1 to about 10 mg per day. Also, in the case of other animals, it is possible to administer an amount converted to 60 kg of body-weight or surface area.

### Brief Description of the Drawings

Figure 1 depicts a photograph demonstrating the result of RT-PCR analysis on the expression of Helicain proteins in various kinds of cells.
Figure 2 depicts a schematic illustration of structures of human Helicain A, B, and C proteins.
Figure 3 depicts the DEAD box of the human Helicain B protein.
Figure 4 depicts the DEAD box of the human Helicain C protein.
Figure 5 depicts a graph demonstrating the result of analysis on the binding region of Tesmin by the yeast two-hybrid method.
Figure 6A depicts a photograph demonstrating the result of analysis wherein the expression of the V5 fused recombinant Helicain B and Helicain C proteins expressed in COS7 cells is detected. Figure 6B depicts photographs demonstrating the result of analysis wherein the expression of the His-tag fused recombinant Helicain A expressed in *E. coli* is detected.
Figure 7 depicts photographs demonstrating the result of analysis by Northern blotting on the expression of Helicains in various tissues and cancer cells.
Figure 8 depicts a drawing demonstrating the location of the human Helicain gene on chromosome.
Figure 9 depicts photographs demonstrating the intracellular localization of the human Helicain proteins.

### Best Mode for Carrying out the Invention

The present invention will be explained in detail below with reference to examples, but is not construed as being limited thereto.

### [Example 1] Detection of Helicains by RT-PCR

Total RNA was extracted from normal human cells (MRC-5, KMS-6, HBL-100, and HUVEC) and cancer cells. After denaturing the RNA at 65°C, cDNAs were prepared using reverse transcriptase: Superscript II (GIBCO/BRL). Using the cDNAs as templates, they were analyzed for Helicain and GAPDH at conditions of 32 cycles of 94°C for 1 minute, 58°C for 2 minutes, and 72°C for 3 minutes using oligo primers, SEQ ID NO: 9 and 10, designed within the common part of Helicain A, B, and C, and 30 cycles of 94°C for 1 minute, 58°C for 2 minutes, 72°C for 3 minutes using oligo primers, SEQ ID NO: 7 and 8, respectively.

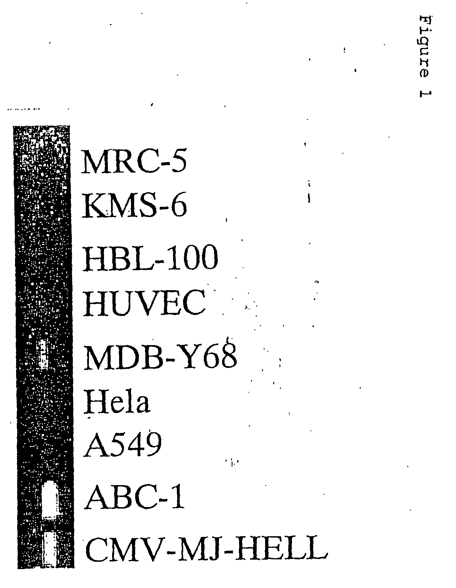

As a result, a strong expression was observed in several cancer cells while only a weak expression could be observed in any of the normal cells (Figure 1).

### [Example 2] Cloning and sequencing of cDNA

The present inventors tried to isolate genes encoding proteins interacting with testis specific protein, Tesmin (Sugihara; T. et al., 1999, Genomics 57: 130-136) by yeast two-hybrid method. As a result, novel gene fragments, Helicains, were obtained. The sequences of these gene fragments were novel, and no sequence homologous with these sequences could be found in DNA sequence databanks. Further, based on the information of the gene fragments, full-length cDNAs were cloned from human thyroid gland derived Marathon Ready™ cDNA by the 5'-Race and 3'-Race methods. 3 antisense primers specific to Helicain genes were used in the methods: i.e., SP1 (SEQ ID NO: 11), AP1 (SEQ ID NO: 12), SP2 (SEQ ID NO: 13), and AP2 (SEQ ID NO: 14)) for the 5'-Race method; and SP1 (SEQ ID NO: 15), AP1 (SEQ ID NO: 12), SP2 (SEQ ID NO: 16), and AP2 (SEQ ID NO: 14) for the 3'-Race method. Human thyroid gland derived Marathon Ready™ cDNA was used as a template. Furthermore, the 5'-Race and 3'-Race methods were carried out according to the manufacturer's protocol described in "Marathon-Ready™ cDNA kit (mouse testis)" (Clontech). The present inventors determined the sequence of the isolated clones, Helicains, by the dideoxy-chain termination method, and analyzed by ABI377 automatic DNA sequencer.

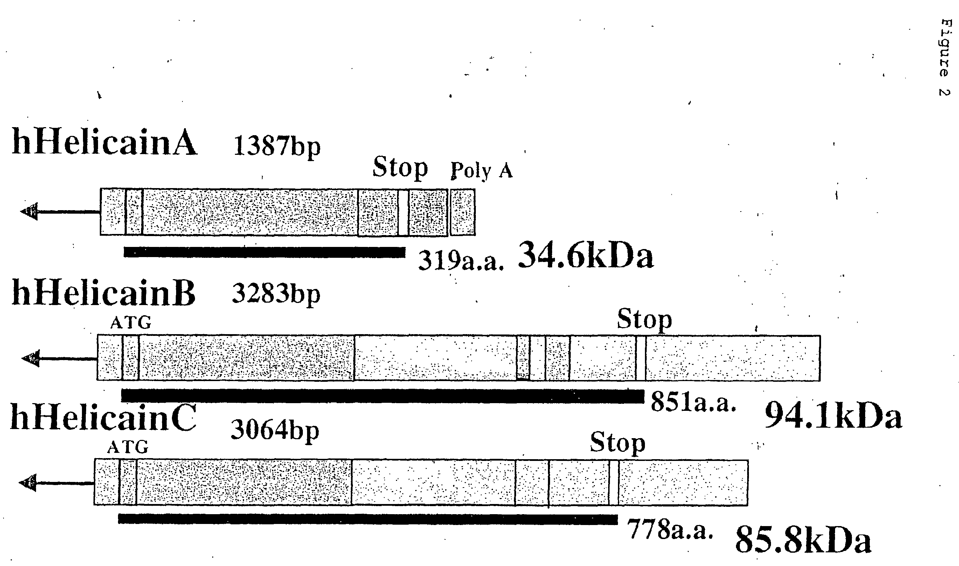

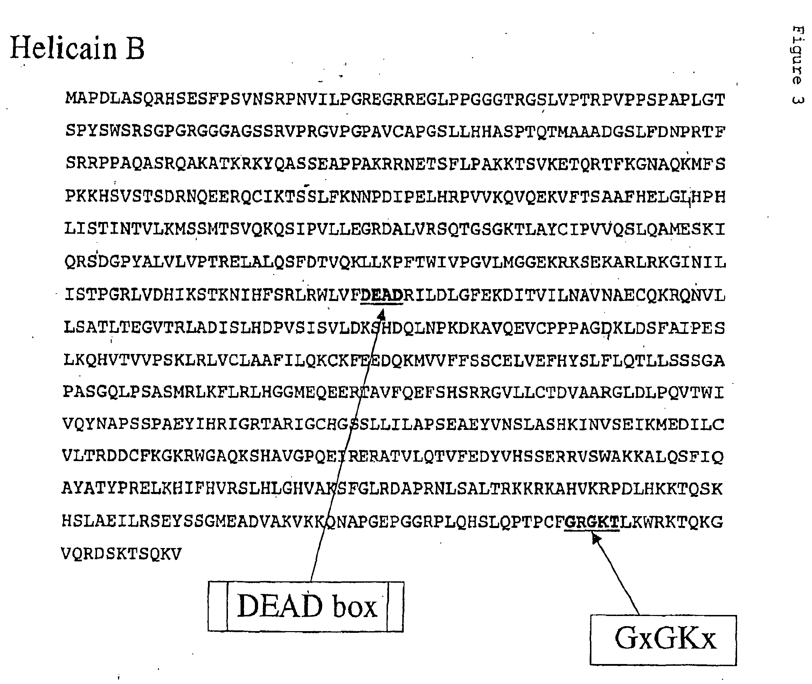

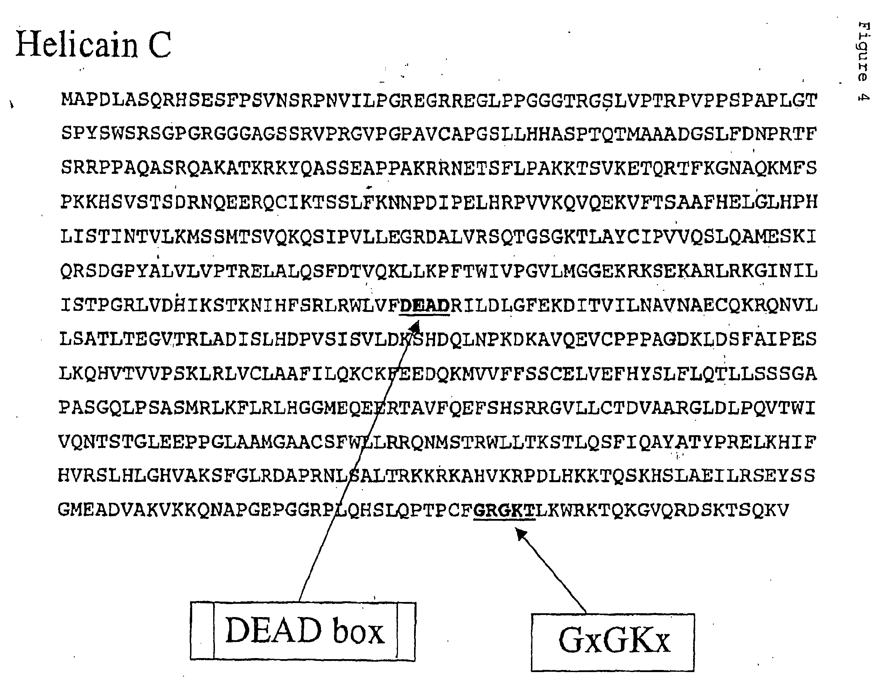

The obtained full-length nucleotide sequences of Helicain cDNAs are shown in Figure 2 and SEQ ID NO: 1 (Helicain A), SEQ ID NO: 3 (Helicain B), and SEQ ID NO: 5 (Helicain C). No sequence homologous to the obtained full-length cDNA sequences could be found in DNA sequence databanks. The obtained cDNAs encoded proteins (pI-11.06, 10.43, and 10.60, respectively) consisting of 319 amino acids (Helicain A: SEQ ID NO: 2), 851 amino acids (Helicain B: SEQ ID NO: 4), and 778 amino acids (Helicain C: SEQ ID NO: 6), respectively. No protein significantly identical to these proteins could be discovered in protein databases. Further, despite no identity could be found between the DNA sequences, human Helicain B and C were revealed to possess a DEAD box structure and a GxGKx sequence (Figure 3 and 4) by a Motif Search of the amino acid sequences. On the other hand, Helicain A was a splicing form lacking a DEAD box structure. Two kinds of DEAD/H box helicases, DNA helicase that react with DNA and RNA helicase that react with RNA, are known to exist.

### [Example 3] Determination of the site of Tesmin to interact with Helicain

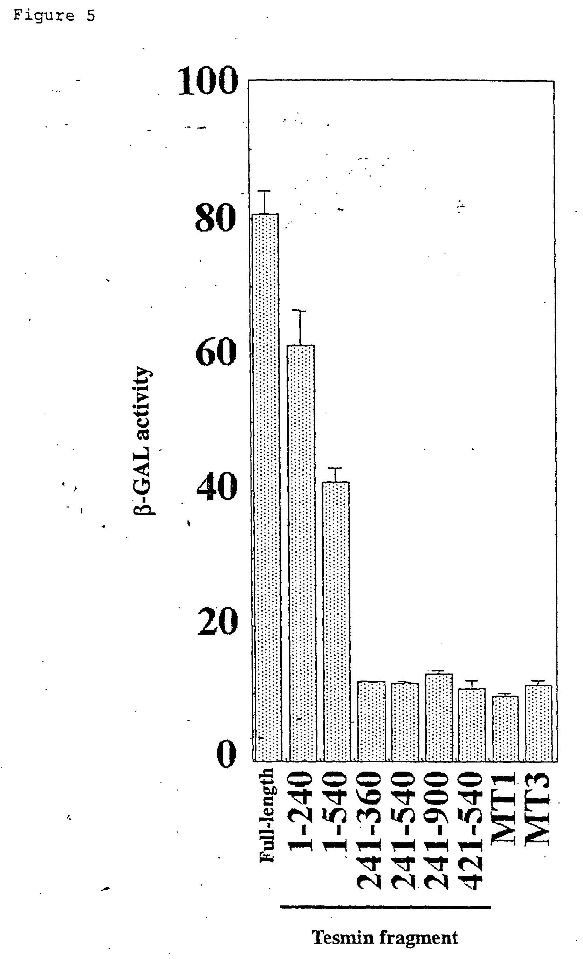

To determine the interaction site of Tesmin, analysis by yeast two-hybrid method was conducted using mutants, wherein a portion of the Tesmin sequence had been deleted. As Helicain protein, a part (amino acids 91 to 319 of the amino acid sequence (SEQ ID NO: 2)) corresponding to the 424bp to 1387bp of Helicain A cDNA (SEQ ID NO: 1), which is a common region among Helicains A, B, and C, was used. As a result, the Helicain binding domain was demonstrated to exist within a region from the 1st to 240th bp in the ORF of Tesmin gene (corresponding to the amino acids 1 to 80 of the protein) (Figure 5). Furthermore, due to the fact that Tesmin has a metallothionein region, the possibility of metallothionein gene products (MT1 and MT3) to interact with Helicain gene products was investigated. However, no interaction could be observed.

### [Example 4] Verification of Helicain by gene transfer

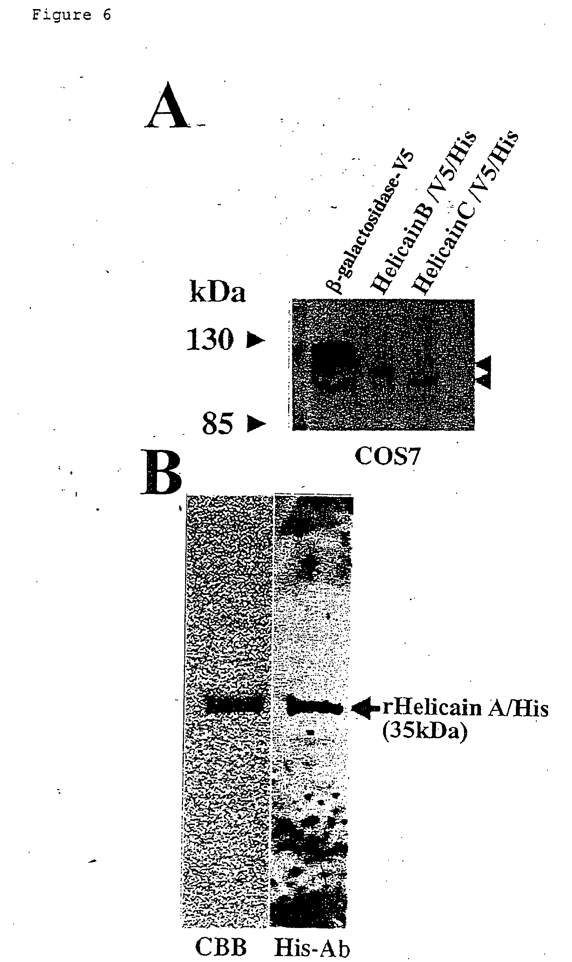

To prove the predicted open reading frame of human Helicains, fusion proteins with V5/His tag was constructed. The open reading frames of Helicain B and C cDNAs were amplified by PCR using sense (SEQ ID NO: 17) and antisense (SEQ ID NO: 18) primers. Specifically, PCR was performed using RT-mix, which had been prepared from HT1080 cells, as a template to conduct cloning. The resultant PCR products were ligated into pcDNA3.1 TOPO Vector (Invitrogen) added with V5-His tag, and were transfected into COS7 cells using Lipofectamine Plus (GIBCO BRL). The cells were lysed with lysis buffer, separated by SDS-PAGE method, and were detected with anti-V5 antibody. As a result, proteins with expected sizes, 96 kDa (Helicain B) and 87 kDa (Helicain C), were detected (Figure 6), which proved coincident with the size of proteins predicted from the ORF in human Helicain sequences.

### [Example 5] Preparation of recombinant Helicain

The open reading frame of Helicain A was amplified by PCR using sense (SEQ ID NO: 17) and antisense (SEQ ID NO: 19) primers comprising *Bam*HI sites. Fragments amplified by PCR were cloned into pGEM-T vector to confirm their accurate sequence. Then the fragments were digested with *Bam*HI and *Sal*I, and were cloned into pQE30 vector that finally produces His fusion proteins. *E. coli* JM109 was transfected with the cloned pQE30 to produce the Helicain, induced for 3 hrs at 37°C using 0.2 mM IPTG, and then the lysate of *E. coli* was separated by SDS-PAGE method to detected the Helicain products by western blotting using anti-His antibody. As a result, a protein about 35 kDa including His-fused region was confirmed to be synthesized (Figure 6B). The size of the recombinant protein was as expected.

### [Example 6] Northern blot analysis

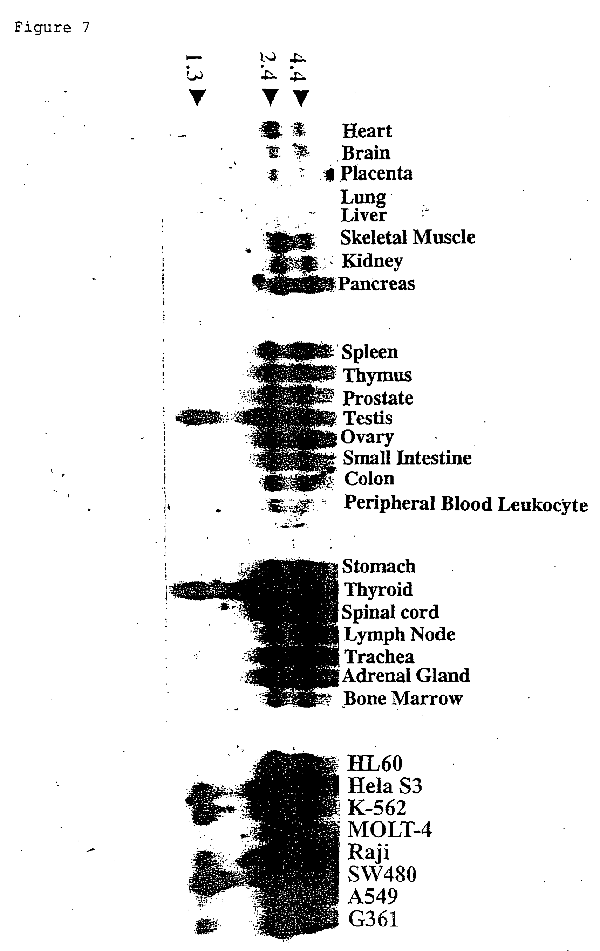

Membrane loaded with 2 µg/lane of mRNA of various human tissues (Clontech laboratories, Palo alto, CA) was purchased to conduct Northern blotting analysis. Gene fragment (566 to 1011 of SEQ ID NO: 3) of Human Helicain was used as a probe. After a prehybridization at 68°C for 30 min using "Rapid-hyb buffer" (Amersham LIFE SCIENCE), labeled probe was added to conduct hybridization by incubating at 68°C for 2 hr. Then, washing three times for 20 min at room temperature in 2x SSC and 0.01% SDS, three times for 20 min at 37°C in 1x SSC and 0.1% SDS, and two times for 20 min at 50°C in 1x SSC and 0.1% SDS, was conducted. The detection was performed by autoradiography. A very strong expression of the gene was observed in thyroid gland, whereas a quite weak expression was observed in other normal tissues. Moreover, a significantly higher expression in several cancer cells compared with normal tissues (except thyroid gland) was detected (Figure 7).

### [Example 7] Chromosomal localization

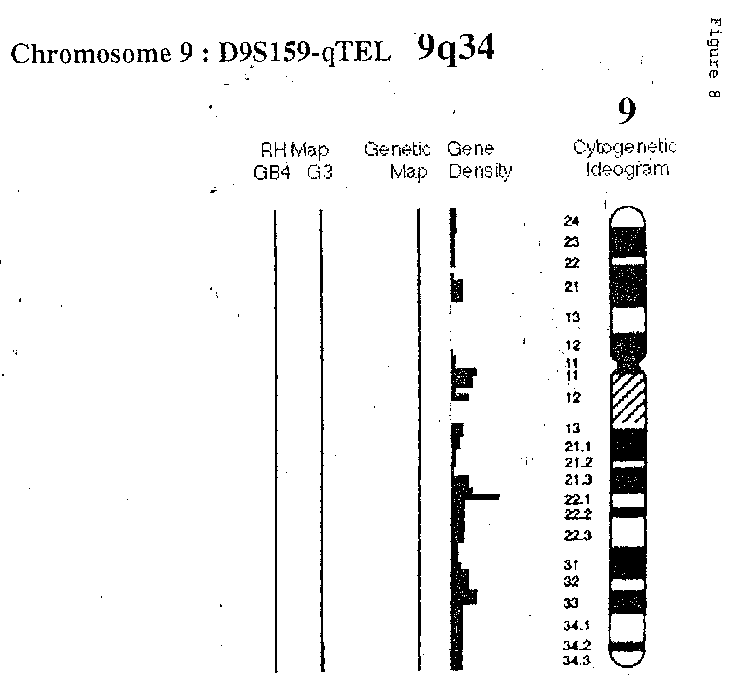

According to EST search in NCBI for genes homologous to the Helicain B sequence, several EST clones were detected. The EST clones were determined to be positioned on 9q34 chromosome by Human UniGene search (Figure 8).

### [Example 8] Intracellular localization

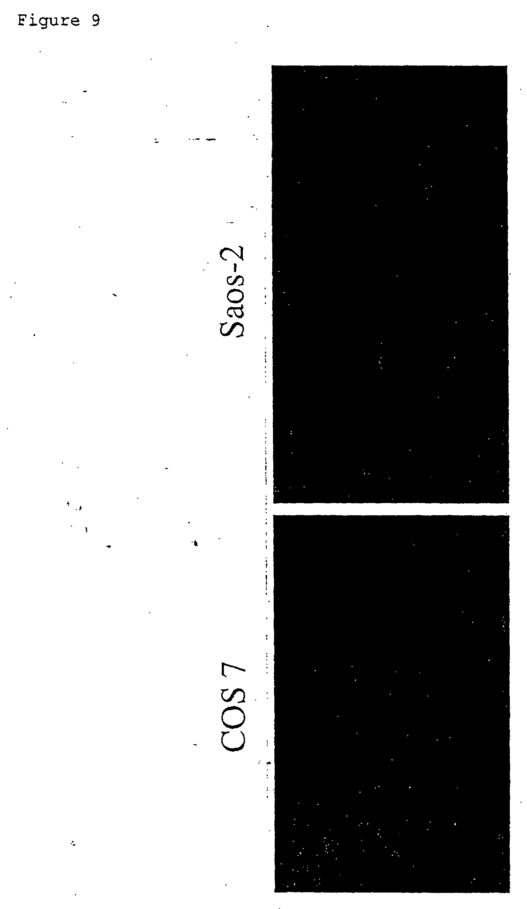

All of the open reading frames of Helicain A cDNA were amplified by PCR using sense (SEQ ID NO: 17). and antisense (SEQ ID NO: 20) primers. These genes were inserted into the C-terminal region of GFP protein in GFP-TOPO Vector (Invitrogen). The resultant plasmid encoding a GFP-Helicain fusion protein was transfected, using Lipofectamine (GIBCO BRL), into COS1 cells that were grown on cover glass. The cover glass was fixed with methanol/acetone (1:1) and washed 3 times with PBS. The cells were observed using Olympus BH2 microscope with epi-fluorescence optical system. As a result, the protein, wherein all of the full-length sequence of Helicain had been fused together, was localized in nucleolus (Figure 9). Similar experiment was conducted with Helicain "B cDNA to confirm the localization in nucleolus.

### Industrial Applicability

The present invention provides novel RNA helicases and genes encoding them. RNA helicases are known to have an enzyme activity to dissociate RNA complex in cells. Recently, RNA helicase is also recognized as factors regulating transcription by forming complex with RNA polymerase, which is a fundamental factor of transcriptional regulation. The present invention provides proteins with a structure similar to RNA helicase, and genes encoding the proteins. The expression of Helicain proteins were demonstrated to be significantly elevated in cancer cells compared with normal tissues except thyroid gland. According to the result, Helicains are expected to be applicable as novel cancer cell markers by detecting the expression levels thereof in cancer cells. Furthermore, localization of Helicains in nucleolus of cells was demonstrated. Therefore, Helicains are expected to be factors strongly involved in the synthesis of ribosomal RNA (rRNA), which is a function of nucleolus. rRNA is closely related to cell proliferation, and the level of rRNA synthesis are elevated in cancer cells due to the active proliferation. Some of the RNA helicases are reported to form a complex with RNA polymerase to regulate the activity of transcription. Due to the possibility of Helicains to regulate rRNA transcription in nucleolus, the development of compounds inhibiting the transcriptional activity of Helicains may serve as a tool for developing anti cancer drugs with a quite novel mechanism for suppressing the growth of cancer cells.

## Claims

1. A DNA selected from the group consisting of:
(a) a DNA encoding a protein comprising the amino acid sequence of SEQ ID NO: 2, 4 or 6; and
(b) a DNA comprising the coding region of the nucleotide sequence of SEQ ID NO: 1, 3 or 5.

2. A DNA encoding a protein having the activity to bind with Tesmin protein selected from the group consisting of:
(a) a DNA encoding a protein comprising the amino acid sequence of SEQ ID NO: 2, 4 or 6 in which one or more amino acids are substituted, deleted, inserted, and/or added; and
(b) a DNA hybridizing with a DNA consisting of the nucleotide sequence of SEQ ID NO: 1, 3 or 5.

3. A DNA encoding a protein having RNA helicase activity selected from the group consisting of:
(a) a DNA encoding a protein comprising the amino acid sequence of SEQ ID NO: 4 or 6 in which one or more amino acids are substituted, deleted, inserted, and/or added; and
(b) a DNA hybridizing with a DNA consisting of the nucleotide sequence of SEQ ID NO: 3 or 5.

4. A protein encoded by the DNA of any one of claims 1 to 3.

5. A vector into which the DNA of any one of claims 1 to 3 is inserted.

6. A host cell retaining the vector of claim 5.

7. A method for producing the protein of claim 4, comprising the steps of: culturing the host cell of claim 6, and recovering the expressed protein from said host cell or the culture supernatant thereof.

8. A partial peptide of the protein of claim 4.

9. A polynucleotide complementary to either a DNA that comprises the nucleotide sequence of SEQ ID NO: 1, 3 or 5, or its complementary strand, wherein -the polynucleotide comprises at least 15 nucleotides.

10. A method of screening for a compound that binds to the protein of claim 4, comprising the steps of:
(a) contacting the protein or partial peptide thereof with a test sample;
(b) detecting the binding activity of the protein or partial peptide thereof with the test sample; and
(c) selecting the compound that binds to the protein or partial peptide thereof.

11. An antibody binding to the protein of claim 4.

12. A compound binding to the protein of claim 4, which is isolated by the method of claim 10.

13. A method of examining for cancer, comprising the step of detecting mRNA that encodes the protein of claim 4 in the test sample.

14. A method of examining for cancer, comprising the step of detecting the protein of claim 4 in the test sample.

15. A reagent for examining for cancer, comprising the antibody of claim 11.

16. A method of screening for a compound that regulates the binding between the protein of claim 4 and Tesmin, comprising the steps of:
(a) contacting the protein of claim 4 with Tesmin in the presence of a test sample;
(b) detecting the binding between the protein of claim 4 and Tesmin; and
(c) selecting the compound that suppresses or promotes the binding compared with that observed in the absence of the test sample.

17. A compound regulating the binding between the protein of claim 4 and Tesmin, which is isolated by the method of claim 16.

18. A method of screening for a compound that regulates RNA helicase activity of a protein comprising the amino acid sequence of SEQ ID NO: 4 or 6, or a protein encoded by the DNA of claim 3, comprising the steps of:
(a) contacting a double stranded nucleic acid as a substrate to the protein in the presence of a test sample;
(b) detecting the dissociation of single strand RNA from the double stranded nucleic acid; and
(c) selecting the compound that suppresses or promotes the dissociation compared with that observed in the absence of the test sample.

19. A compound regulating RNA helicase activity of a protein comprising the amino acid sequence of SEQ ID NO: 4 or 6, or a protein encoded by the DNA of claim 3, wherein the compound is isolated by the method of claim 18.

20. A method of screening for a compound that regulates the localization into the nucleolus of the protein of claim 4, comprising the steps of:
(a) contacting a test sample with or introducing a test sample into cells expressing the protein;
(b) detecting the cellular localization of the protein; and
(c) selecting the compound that suppresses or promotes the localization of the protein into the nucleolus compared with that observed in the absence of the test sample.

21. A compound regulating the nuclear localization of the protein of claim 4, wherein the compound is isolated by the method of claim 20.

22. A pharmaceutical composition comprising the compound of claim 12, 17, 19 or 21 as the active ingredient.

23. An anticancer agent comprising, as the active ingredient, a compound that suppresses RNA helicase activity of a protein comprising the amino acid sequence of SEQ ID NO: 4 or 6, or the protein encoded by the DNA of claim 3, which compound is isolated by the method of claim 18; or a compound suppressing the localization into nucleolus of the protein of claim 4, which compound is isolated by the method of claim 20.
